(19) Europäisches Patentamt
European Patent Office
Office européen des brevets

(11) EP 3 805 212 A1

(12) EUROPEAN PATENT APPLICATION
published in accordance with Art. 153(4) EPC

(43) Date of publication:
**14.04.2021 Bulletin 2021/15**

(21) Application number: **19807362.9**

(22) Date of filing: **09.05.2019**

(51) Int Cl.:
***C07D 401/08*** *(2006.01)* ***A61K 31/4709*** *(2006.01)*
***A61P 35/00*** *(2006.01)*

(86) International application number:
**PCT/CN2019/086258**

(87) International publication number:
**WO 2019/223548 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.05.2018 CN 201810500290**

(71) Applicant: **Beijing InnoCare Pharma Tech Co., Ltd.**
**Beijing 102206 (CN)**

(72) Inventors:
- **CHEN, Xiangyang**
  **Beijing 102206 (CN)**
- **PANG, Yucheng**
  **Beijing 102206 (CN)**

(74) Representative: **Patentanwälte Magenbauer & Kollegen**
**Partnerschaft mbB**
**Plochinger Straße 109**
**73730 Esslingen (DE)**

# (54) 3-OXAZOLINONE COMPOUND, PREPARATION METHOD THEREFOR AND PHARMACEUTICAL APPLICATION THEREOF

(57) The present invention relates to a novel 3-indazolinone compound that regulates or inhibits the activity of indoleamine 2,3-dioxygenase (IDO), the method for the preparation thereof and the use thereof in medicine. In particular, the present invention relates to a compound represented by the general formula (I) and a pharmaceutically acceptable salt thereof, a pharmaceutical composition comprising the compound or a pharmaceutically acceptable salt thereof, a method for treating or preventing IDO-mediated diseases, especially tumors, by use of the compound or a pharmaceutically acceptable salt thereof, and a method for preparing the compound or a pharmaceutically acceptable salt thereof. The present invention further relates to use of the compound or a pharmaceutically acceptable salt thereof or a composition comprising the compound or a pharmaceutically acceptable salt thereof in the manufacture of a medicament for treating or preventing IDO-mediated diseases, especially tumors.Wherein the substituents of the general formula (I) are defined the same as that in the specification.

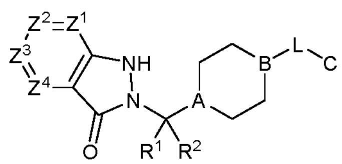

(I)

EP 3 805 212 A1

## Description

### Technical Field

The present invention relates to a novel 3-indazolinone compound or a pharmaceutically acceptable salt thereof that regulates or inhibits the activity of indoleamine 2,3-dioxygenase (IDO), a pharmaceutical composition comprising the compound or a pharmaceutically acceptable salt thereof, a method for the preparation of the same and use of the compound or a pharmaceutically acceptable salt thereof or a pharmaceutical composition comprising the compound or a pharmaceutically acceptable salt thereof in the manufacture of a medicament for treating and/or preventing IDO-mediated related disorders, especially tumors, and the usage thereof.

### Background Art

Indoleamine 2,3-dioxygenase (IDO) is a monomeric protein containing heme which is widely distributed in tissues other than the liver. The IDO catalyzes the oxidative degradation of tryptophan into kynurenine, and is the rate-limiting enzyme in the metabolic pathway of kynurenine. Tryptophan is an essential amino acid for the proliferation of T cells, and it is also a precursor for the synthesis of neurotransmitters. If the concentration of tryptophan in the cell microenvironment decreases and the level of kynurenine increases, it will cause T cells to stagnate in the G1 metaphase, which will affect the proliferation, differentiation and activity of T cells. IDO is expressed at a low level in normal cells, but is overexpressed in many tumor tissues, leading to abnormal local tryptophan metabolism and the formation of regulatory T cells, which in turn mediates local T cell immune tolerance in the tumor. IDO plays an important role in the process of occurrence, development and transfer of malignant tumors. If the activity of IDO is inhibited, tryptophan metabolism around tumor cells can be effectively prevented, which can promote the growth of T cells, thereby enhancing the body's immune system to fight tumors. Therefore, the development of IDO inhibitors has become a frontier hot spot in tumor immunotherapy drug research. Preclinical studies have shown that a single administration of INCB-024360, a selective inhibitor of IDO, can effectively inhibit the plasma IDO activity of blank mice to be at the level of IDO-deficient mice, and the repeated administration of INCB-024360 hinders the expansion of CT26 tumors.(Koblish et. al, Mol. Cancer Ther. 2010, 9, 489-98).

IDO inhibitors can also be combined with other anti-tumor small molecule drugs and immune checkpoint inhibitors, such as CTLA-4, PD-1 and PD-L1 antibodies, to enhance the anti-tumor efficacy of the drug. The combined immunotherapy of small molecule IDO inhibitors and immune checkpoint inhibitors, such as indoximod/ipilimumab, epacadostat/pembrolizumab, epacadostat/nivolumab, indoximod/MEDI-4736, etc., is in clinical trials. Preliminary clinical results show that the combination of IDO small molecule inhibitors and PD-1 has additional effects, achieves good disease control rates in the treatment of various tumors, and has fewer side effects than PD-1/CTLA-4, showing a broad prospects for tumor immunotherapy(AACR, 2017; ASCO, 2017).

In addition to cancer, IDO is also associated with many other diseases, such as immunosuppression, chronic infections, viral infections, autoimmune diseases or disorders (such as rheumatoid arthritis), neurological or neuropsychiatric diseases or disorders (such as depression), etc. Therefore, IDO inhibitors are of great therapeutic value.

Small molecule IDO inhibitor drugs are still in clinical trials now. In addition to Incyte's INCB-024360 (epacadostat), examples can also be found in indoximod from NewLink Genetics and BMS-986205 from Bristol-Myers Squibb.

The development of IDO inhibitors has attracted the attention of many biopharmaceutical companies due to its prospects shown in the single and combined immunotherapy of various tumors and other diseases. A series of patent applications for IDO inhibitors have been published, including WO2006122150A1, WO2011056652A1, WO2013069765A1, WO2014186035A1, WO2015002918A1, WO2016073738A2, WO2016073770A1, WO2016181348A1, WO2016161960A1, WO2017079669A1 etc. However, there is still a need to develop new compounds with better druggability and higher response rates in immunotherapy. After continuous efforts, the present inventors designed a compound having a structure represented by the general formula (I), and found that the compound of such structure exhibits excellent effect in inhibiting IDO activity.

### Description of the Invention

The present invention provides a compound represented by general formula (I) :

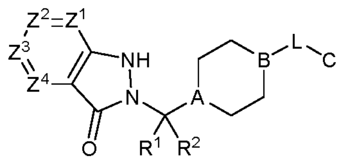

(I)

or a pharmaceutically acceptable salt, prodrug, stable isotope derivative, isomer thereof and mixture of the same, which acts as an IDO inhibitor, wherein:

$Z^1$, $Z^2$, $Z^3$ and $Z^4$ are each independently selected from N or $CR^3$, but $Z^1$, $Z^2$, $Z^3$ and $Z^4$ cannot be N at the same time;

$R^1$ and $R^2$ are each independently selected from H , or optionally substituted $C_{1-4}$ alkyl,$C_{3-6}$ cycloalkyl or 4-7 membered heterocyclyl; or, $R^1$ and $R^2$, together with the carbon atom to which they are attached, optionally form a 3-7 membered ring optionally comprising a heteroatom selected from O, N and S;

A is N or $CR^4$;

B is N or $CR^5$;

L is a bond, -O- or $-CR^6R^7-$;

C is an optionally substituted 4-7 membered heterocyclyl, 6-10 membered aryl or 5-10 membered heteroaryl;

$R^3$ is independently selected from H, halogen, cyano, $-SF_5$, -OR, -SR, $-NR_2$, $-S(O)_mR$, $-S(O)_2NR_2$, $-N(R)S(O)_2R$, $-C(O)NR_2$, -N(R)C(O)R, or optionally substituted $C_{1-4}$ alkyl, $C_{3-6}$ cycloalkyl, 4-7 membered heterocyclyl, phenyl or 5-6 membered heteroaryl;

$R^4$ and $R^5$ are each independently selected from H, halogen, OH, or optionally substituted $C_{1-4}$ alkyl or $-O-C_{1-4}$ alkyl;

$R^6$ and $R^7$ are each independently selected from H or optionally substituted $C_{1-4}$alkyl;

R is independently selected from H or optionally substituted $C_{1-4}$ alkyl, $C_{3-6}$ cycloalkyl, 4-7 membered heterocyclyl, phenyl or 5-6 membered heteroaryl; the two Rs on the same nitrogen atom together with the nitrogen atom to which they are attached optionally form a 4-7 membered heterocyclic ring, optionally containing additional heteroatom(s) selected from O, N and S;

m is 1 or 2.

In one embodiment, the present invention relates to the compound represented by the above general formula (I) or a pharmaceutically acceptable salt, prodrug, stable isotope derivative, isomer thereof and mixture of the same, wherein:

$Z^1$, $Z^2$, $Z^3$ and $Z^4$ are each independently selected from N or $CR^3$, but at most one of $Z^1$, $Z^2$, $Z^3$ and $Z^4$ is N;

$R^1$ and $R^2$ are each independently selected from H or optionally substituted $C_{1-4}$alkyl;

A is N or $CR^4$;

B is N or $CR^5$;

L is a bond or -O-;

C is an optionally substituted 4-7 membered heterocyclyl, 6-10 membered aryl or 5-10 membered heteroaryl, wherein the substitution is selected from halogen, cyano, $C_{1-4}$ alkyl or halogenated $C_{1-4}$ alkyl;

$R^3$ is independently selected from H, halogen, cyano, $-SF_5$, -OR, -SR, $-NR_2$, $-S(O)_mR$, $-S(O)_2NR_2$, $-N(R)S(O)_2R$, $-C(O)NR_2$, -N(R)C(O)R, or optionally substituted $C_{1-4}$ alkyl, $C_{3-6}$ cycloalkyl, 4-7 membered heterocyclyl, phenyl or

5-6 membered heteroaryl;

$R^4$ and $R^5$ are each independently selected from H, halogen, OH, $C_{1-4}$ alkyl or -O-$C_{1-4}$ alkyl;

R is independently selected from H or optionally substituted $C_{1-4}$ alkyl, $C_{3-6}$ cycloalkyl, 4-7 membered heterocyclyl, phenyl or 5-6 membered heteroaryl; the two Rs on the same nitrogen atom together with the nitrogen atom to which they are attached optionally form a 4-7 membered heterocyclic ring, said 4-7 membered heterocyclic ring optionally containing additional heteroatom(s) selected from O, N and S;

m is 1 or 2.

In another embodiment, the present invention relates to the compound or a pharmaceutically acceptable salt prodrug, stable isotope derivative, isomers thereof and mixture of the same according to any one of the above embodiments , wherein:

$Z^1$, $Z^2$, $Z^3$ and $Z^4$ are each independently selected from N or $CR^3$, but at most one of $Z^1$, $Z^2$, $Z^3$ and $Z^4$ is N;

$R^1$ and $R^2$ are each independently selected from H or $C_{1-4}$ alkyl;

A is N or CH;

B is N or CH;

L is a bond;

C is a 5-10 membered heteroaryl optionally substituted by halogen, cyano, $C_{1-4}$ alkyl or halogenated $C_{1-4}$ alkyl;

$R^3$ is independently selected from H, halogen, cyano, or optionally substituted $C_{1-4}$ alkyl, -O-$C_{1-4}$ alkyl, $C_{3-6}$ cycloalkyl or 4-7 membered heterocyclic group;

the "optionally substituted" means substitution by a substituent selected from the group consisting of halogen, -CN, -OR’, -NR’R", wherein R’ and R" are each independently selected from H, $C_{1-4}$ alkyl or $C_{3-7}$ cycloalkyl.

In another embodiment, the present invention relates to the compound or a pharmaceutically acceptable salt, prodrug, stable isotope derivative, isomer thereof and mixture of the same according to any one of the above embodiments, wherein:

$Z^1$ and $Z^4$ are each independently selected from N or CH, $Z^2$ and $Z^3$ are each independently selected from N or $CR^3$, but at most one of $Z^1$, $Z^2$, $Z^3$ and $Z^4$ is N;

$R^1$ and $R^2$ are each independently selected from H or $C_{1-4}$alkyl;

A is CH;

B is CH;

L is a bond;

C is a 5-10 membered heteroaryl optionally substituted by halogen or $C_{1-4}$

alkyl;

$R^3$ is H, halogen or cyano.

In another embodiment, the present invention relates to the compound according to any one of the above embodiments, wherein:
C is quinolinyl or pyridyl optionally substituted by halogen or $C_{1-4}$ alkyl, especially fluoroquinolinyl.

In another embodiment, the present invention relates to the compound according to any one of the above

embodiments, wherein:

$R^1$ is $C_{1-4}$ alkyl, $R^2$ is H, especially $R^1$ is methyl and $R^2$ is H.

In another embodiment, the present invention relates to the compound according to any one of the above embodiments, wherein:

the compound is represented by the following general formula (IIa)-(IIc)

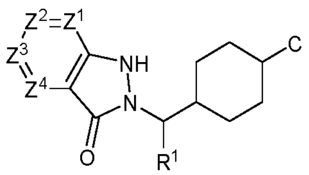
(IIa)
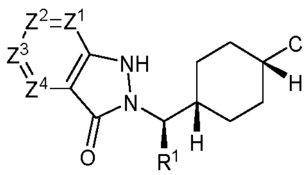
(IIb)
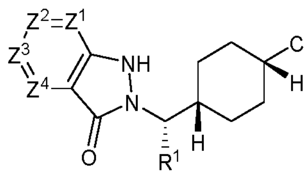
(IIc) .

In another embodiment, the present invention relates to the compound according to any one of the above embodiments, wherein:

the compound is represented by the following general formula (III)

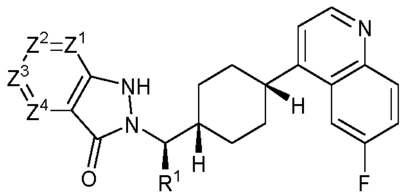
(III) .

In another embodiment, the present invention relates to the compound represented by the above general formula (III), wherein $R^1$ is methyl.

In another embodiment, the present invention relates to the compound represented by the above general formula (I), wherein the compound is selected from:

| Number of the compound | Structure and name of the compound |
|---|---|
| 1. | 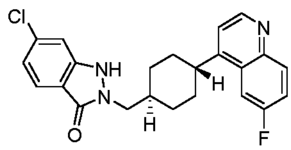 |
| | 6-chloro-2-(((1r,4r)-4-(6-fluoroquinolin-4-yl)cyclohexyl)methyl)-1,2-di hydro-3H-indazol-3-one |
| 2. | 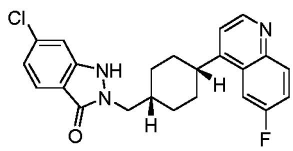 |
| | 6-chloro-2-(((1s,4s)-4-(6-fluoroquinolin-4-yl)cyclohexyl)methyl)-1,2-d ihydro-3H-indazol-3-one **2** |
| 3. | 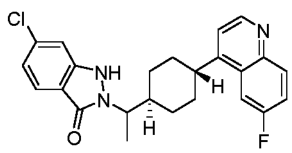 |
| | 6-chloro-2-(1-((1r,4r)-4-(6-fluoroquinolin-4-yl)cyclohexyl)ethyl)-1,2-d ihydro-3H-indazol-3-one |

(continued)

| Number of the compound | Structure and name of the compound |
|---|---|
| 4. | 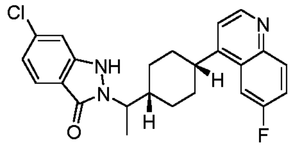<br>6-chloro-2-(1-((1s,4s)-4-(6-fluoroquinolin-4-yl)cyclohexyl)ethyl)-1,2-d ihydro-3H-indazol-3-one |
| 5. | 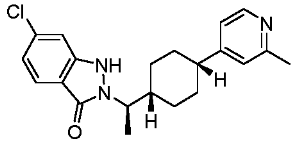<br>6-chloro-2-((R)-1-((1s,4S)-4-(2-methylpyridin-4-yl)cyclohexyl)ethyl)-1,2-dihydro-3H-indazol-3-one |
| 6. | 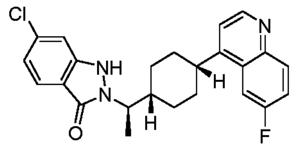<br>6-chloro-2-((R)-1-((1s,4S)-4-(6-fluoroquinolin-4-yl)cyclohexyl)ethyl)-1,2-dihydro-3H-indazol-3-one |
| 7. | 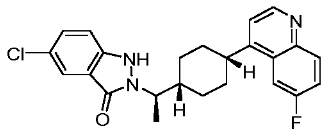<br>5-chloro-2-((R)-1-((1s,4S)-4-(6-fluoroquinolin-4-yl)cyclohexyl)ethyl)-1,2-dihydro-3H-indazol-3-one |
| 8. | 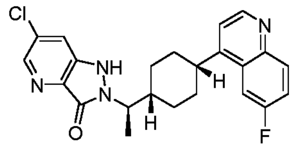<br>6-chloro-2-((R)-1-((1s,4S)-4-(6-fluoroquinolin-4-yl)cyclohexyl)ethyl)-1,2-dihydro-3H-pyrazolo[4,3-b] pyridin-3-one |
| 9. | 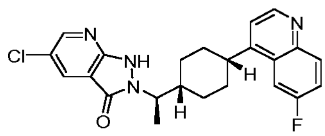<br>5-chloro-2-((R)-1-((1s,4S)-4-(6-fluoroquinolin-4-yl)cyclohexyl)ethyl)-1,2-dihydro-3H-pyrazolo[3,4-b] pyridin-3-one |
| 10. | 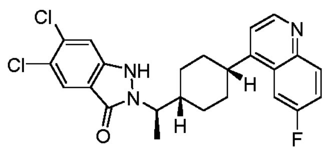<br>5,6-dichloro-2-((R)-1-((1s,4 S)-4-(6-fluoroquinolin-4-yl)cyclohexyl)eth yl)-1,2-dihydro-3H-indazol-3-one |

(continued)

| Number of the compound | Structure and name of the compound |
|---|---|
| 11. | 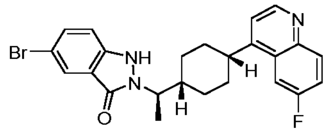 |
| | 5-bromo-2-((R)-1-((1s,4S)-4-(6-fluoroquinolin-4-yl)cyclohexyl)ethyl)-1,2-dihydro-3H-indazol-3-one |
| 12. | 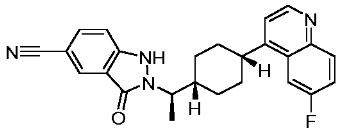 |
| | 2-((R)-1-((1s,4S)-4-(6-fluoroquinolin-4-yl)cyclohexyl)ethyl)-3-oxo-2,3 -dihydro-1H-indazole-5-carbonitrile |

or a pharmaceutically acceptable salt, prodrug, stable isotope derivative, isomer thereof and mixture of the same.

The compound of the present invention has a significant inhibitory effect on the activity of IDO in Hela cells, preferably it has an $IC_{50}$ of less than 200 nM, more preferably it has an $IC_{50}$ of less than 50 nM.

Therefore, the compounds of the present invention can be used to treat or prevent IDO-mediated related diseases, including but not limited to cancer, immunosuppression, chronic infection, viral infection, autoimmune disease or condition (such as rheumatoid arthritis), neurological or neuropsychiatric disease or condition (such as depression), etc. The compounds of the present invention are used to treat or prevent IDO-related tumors,including but not limited to prostate cancer, colon cancer, rectal cancer, pancreatic cancer, cervical cancer, stomach cancer, endometrial cancer, brain cancer, liver cancer, bladder cancer, ovarian cancer, testicular cancer, head cancer, neck cancer, skin cancer (including melanoma and basal cancer), mesothelial and endothelial carcinoma, lymphoma, leukemia, esophageal cancer, breast cancer, muscle cancer, connective tissue cancer, lung cancer (including small cell lung cancer and non-small cell cancer), adrenal cancer, thyroid cancer, kidney cancer, bone cancer, glioblastoma, mesothelioma, sarcoma (including Kaposi's sarcoma), choriocarcinoma, skin basal cell carcinoma or testicular seminoma, etc. Thus, in another aspect, the present invention provides a method for treating or preventing IDO-mediated diseases (such as the tumor), which comprises administering to a patient in need a therapeutically effective amount of the compound of the present invention or a pharmaceutically acceptable salt, prodrug, stable isotope derivative, isomer thereof and mixture of the same, or a pharmaceutical composition containing the compound.

In another aspect, the present invention relates to a compound represented by the general formula (I) or a pharmaceutically acceptable salt, prodrug, stable isotope derivative, isomer thereof and mixture of the same for use as medicines or for medical purposes, which is for use in the treatment or prevention of IDO-mediated related diseases , such as cancer, immunosuppression, chronic infection, viral infection, autoimmune disease or condition (such as rheumatoid arthritis), neurological or neuropsychiatric disease or condition (such as depression), etc.

The present invention further relates to a pharmaceutical composition comprising the compound of the present invention or a pharmaceutically acceptable salt, prodrug, stable isotope derivative, isomer thereof and mixture of the same, and a pharmaceutically acceptable carrier and excipient.

Another aspect of the present invention relates to use of the compound represented by the general formula (I) or a pharmaceutically acceptable salt, prodrug, stable isotope derivative, isomer thereof and mixture of the same, or the pharmaceutical composition in the manufacture of a medicament, wherein the medicament is used for treating or preventing IDO-mediated diseases, such as tumors and immunosuppression.

Another aspect of the present invention relates to a pharmaceutical composition, which comprises the compound represented by the general formula (I) or a pharmaceutically acceptable salt, prodrug, stable isotope derivative, isomer thereof and mixture of the same, and at least one additional medicament, wherein the at least one additional medicament is a chemotherapeutic agent, an immuno and/or inflammatory modulator(such as an immune checkpoint inhibitor), nerve-related disease modulators or an anti-infective.

According to the present invention, the medicament can be in any pharmaceutical dosage form, including but not limited to tablets, capsules, solutions, freeze-dried preparations, and injections.

The pharmaceutical preparation of the present invention can be administered in the form of a dosage unit

containing a predetermined amount of active ingredient per dosage unit. Such dosage unit may contain, for example, from 0.5 mg to 1 g, preferably from 1 mg to 700 mg, particularly preferably from 5 mg to 300 mg of the compound of the present invention based on the condition to be treated, the way of administration, and the age, weight and general condition of the patient. Preferred dosage unit formulations are those containing the active ingredient in daily or divided doses or corresponding fractions thereof as indicated above. In addition, this type of pharmaceutical preparation can be prepared by use of methods known in the pharmaceutical field.

The pharmaceutical preparations of the present invention may be administration by any desired method, such as oral (including oral or sublingual), rectal, nasal, topical (including buccal, sublingual or transdermal), vaginal or parenteral (including subcutaneous, intramuscular, intravenous or intradermal). All methods known in the pharmaceutical field can be used to prepare such formulations by, for example, combining the active ingredient with one or more excipients or one or more adjuvants.

**Embodiments**

Unless stated to the contrary, the following terms used in the specification and claims have the following meanings.

The expression "$C_{x-y}$" as used herein represents the range of the number of carbon atoms, where both x and y are integers. For example, $C_{3-8}$ cycloalkyl represents a cycloalkyl group having 3 to 8 carbon atoms. i.e. cycloalkyl group having 3, 4, 5, 6, 7 or 8 carbon atoms. It should also be understood that "$C_{3-8}$" also includes any sub-range therein, such as $C_{3-7}$, $C_{3-6}$, $C_{4-7}$, $C_{4-6}$, $C_{5-6}$ and the like.

The term "alkyl" refers to a saturated aliphatic hydrocarbon group, including linear and branched groups having 1 to 20 carbon atoms, for example, linear and branched groups having 1 to 8 carbon atoms, 1 to 6 carbon atoms or 1 to 4 carbon atoms. Non-limiting examples include methyl, ethyl, n-propyl, i-propyl, n-butyl, i-butyl, t-butyl, s-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl and 2-ethylbutyl. The alkyl can be optionally substituted.

The term "cycloalkyl" refers to saturated cyclic hydrocarbon groups comprising 3 to 14 cyclic carbon atoms. The cycloalkyl can be monocyclic, and comprises in general 3 to 8, 3 to 7 or 3 to 6 cyclic carbon atoms. Non-limiting examples of monocyclic cycloalkyl include cyclopropyl, cyclobutyl, cyclopentyl, cyclohexyl, and cycloheptyl. The cycloalkyl can optionally be bi- or tricyclic rings fused together, such as decalinyl. The cycloalkyl group can be optionally substituted.

The term "heterocyclyl or heterocycle" refers to a saturated or partially unsaturated monocyclic or polycyclic group, comprising 3 to 20 ring atoms, such as 3 to 14, 3 to 12, 3 to 10, 3 to 8 , 3 to 6 or 5 to 6 ring atoms, where one or more ring atoms are selected from nitrogen, oxygen or $S(O)_m$ (where m is an integer of 0 to 2), but excluding ring parts of -O-O-, -O-S- or -S-S-, and the remaining ring atoms are carbon. Preferably 3 to 12 ring atoms, more preferably 3 to 10 atoms, even more preferably 4 to 7 atoms, most preferably 5 or 6 atoms are comprised; of which 1 to 4 atoms are heteroatoms, more preferably 1 to 3 atoms are heteroatoms, and most preferably 1 to 2 atoms are heteroatoms. Non-limiting examples of monocyclic heterocyclyl include pyrrolidinyl, piperidyl, piperazinyl, pyranyl, morpholinyl, thiomorpholinyl, homopiperazinyl, oxetanyl and azetidinyl. Polycyclic heterocyclic groups include spirocyclic, fused and bridged cyclic heterocyclyl groups, such as octahydrocyclopenta[c]pyrrole, octahydropyrrolo[1,2-a]pyrazine, 3,8-diazabicyclo[3.2.1]octane, 5-azaspiro[2.4]Heptane, 2-oxa-7-azaspiro[3.5]nonane and so on. The heterocyclic group or heterocyclic ring can be optionally substituted.

The term "aryl or aromatic ring" refers to an aromatic monocyclic or condensed polycyclic group comprising 6 to 14 carbon atoms, preferably 6 to 10 membered, such as phenyl and naphthyl, and phenyl is the most preferred. The aryl ring may be fused to a heteroaryl, a heterocyclyl or a cyclic ring, in which the ring connected with the parent structure is an aryl ring, and the non-limiting examples include:

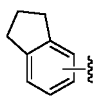 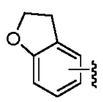 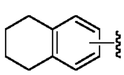 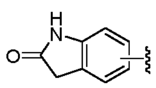 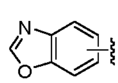 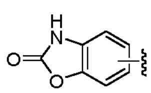 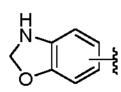

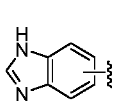 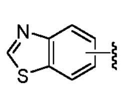 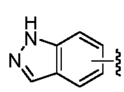 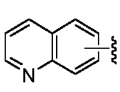 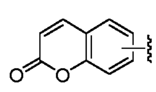

and

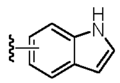,

The aryl or aromatic ring can be optionally substituted.

The term "heteroaryl or heteroaromatic ring" refers to a heteroaromatic system comprising 5 to 14 ring atoms, where 1 to 4 ring atoms are selected from heteroatoms including oxygen, sulfur and nitrogen. Heteroaryl is preferably 5 to 10 membered, and more preferably 5 membered or 6 membered, e.g., furyl, thienyl, pyridyl, pyrrolyl, N-alkylpyrrolyl, pyrimidinyl, pyrazinyl, pyrazolyl, imidazolyl, tetrazyl, oxazolyl, isoxazolyl, thiazolyl, isothiazolyl, quinolinyl etc. The heteroaryl ring can be fused to an aryl, a heterocyclyl or a cycloalkyl ring, where the ring connected with the parent structure is a heteroaryl ring, and the non-limiting examples include:

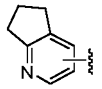 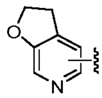 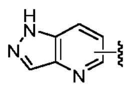 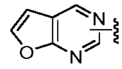 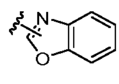 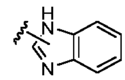

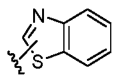 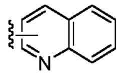

and

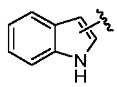,

The heteroaryl or heteroaromatic ring may be optionally substituted.

The term "halogen" refers to fluorine, chloine, bromine or iodine.

The term "cyano" refers to -CN.

The "optional" and "optionally" means that an event or environment described subsequently may but does not necessarily occur, including cases where the event or environment occurs or does not occur. For example, "heterocyclyl optionally substituted by alkyl" means that alkyl may but does not necessarily exist, including cases where heterocyclyl is substituted by alkyl and not substituted by alkyl.

The "optionally substituted" means that one or more hydrogen atoms, preferably at most 5 and more preferably 1 to 3 hydrogen atoms, in a group are substituted independently with corresponding number of substituents. It goes without saying that, substituents are only located in their possible chemical positions, and a person skilled in the art can determine (experimentally or theoretically) possible or impossible substitutions without a lot of efforts. For example, amino or hydroxy groups having free hydrogen may be unstable when combined with carbon atoms having unsaturated (e.g. olefinic) bonds. The substituents include but not limited to halogen, cyano, nitro, oxo, $-SF_5$, $C_{1-4}$alkyl, $C_{3-7}$cycloalkyl, 4-7membered heterocyclic group, phenyl, 5-6membered heteroaryl, -OR', -NR'R", -C(O)R', -C(O)OR', -C(O)NR'R", -C(O)N(R')OR", -OC(O)R', -OC(O)NR'R", -N(R')C(O)OR", -N(R')C(O)R", -N(R''')C(O)NR'R", $-N(R')S(O)_2R"$, $-S(O)_mR'$(m is 1 or 2), $-S(O)_2NR'R"$ etc., wherein said alkyl, cycloalkyl, heterocyclyl, phenyl or heteroaryl is optionally substituted by one or more substitutions selected from the group consisting of halogen, cyano, $C_{1-4}$alkyl, $C_{3-7}$cycloalkyl, 4-7membered heterocyclic group, phenyl, 5-6membered heteroaryl, OR', -NR'R", -C(O)R', -C(O)OR', -C(O)NR'R", -OC(O)NR'R", -N(R')C(O)OR", -N(R')C(O)R", -N(R''')C(O)NR'R", $-N(R')S(O)_2R"$, $-S(O)_2R'$, $-S(O)_2NR'R"$ etc. R', R"and R''' are each independently selected from H, $C_{1-4}$ alkyl optionally containing heteroatoms selected from N, O and S, $C_{3-7}$ cycloalkyl, 4-7 membered heterocyclic group , phenyl or 5-6 membered heteroaryl, wherein the alkyl, cycloalkyl, heterocyclyl, phenyl or heteroaryl is optionally substituted by one or more substitutions selected from the group consisting of halogen, cyano, C1-4 alkyl, halogenated C1-4 alkyl, $-O-C_{1-4}$ alkyl etc; R' and R"on the same nitrogen atom are optionally combined with the nitrogen atom to which they are connected to form a 4-7 membered heterocycle optionally containing additional heteroatom selected from O, S and N.

The term "isomers" refer to compounds that have the same molecular formula but differ in the form or order of their atoms, or the arrangement of their atoms in space. Isomers that differ in the arrangement of their atoms in space are called "stereoisomers". Stereoisomers include optical isomers, geometric isomers and conformational isomers.

The compounds of the present invention may exist in the form of optical isomers. Depending on the configuration of the substituents around the chiral carbon atom, these optical isomers are in the "R" or "S" configuration. Optical isomers include enantiomers and diastereomers. Methods of preparing and separating optical isomers are known in the

art.

The compounds of the present invention may also exist in geometric isomers. The present invention considers various geometric isomers and mixtures thereof resulting from the distribution of substituents around carbon-carbon double bonds, carbon-nitrogen double bonds, cycloalkyl or heterocyclic groups. Substituents around a carbon-carbon double bond or carbon-nitrogen bond are designated as Z or E configuration, and substituents around a cycloalkyl or heterocyclic ring are designated as cis or trans configuration.

The compounds of the present invention may also exhibit tautomerism, such as keto-enol tautomerism.

It should be understood that the present invention includes any tautomeric or stereoisomeric forms and mixtures thereof, and is not limited to any one of the tautomeric or stereoisomeric forms used in the naming or chemical structure of the compound.

"Isotopes" include all isotopes of atoms that appear in the compounds of the present invention. Isotopes include those atoms that have the same atomic number but different mass numbers. Examples of isotopes suitable for incorporation into the compounds of the present invention are hydrogen, carbon, nitrogen, oxygen, phosphorus, fluorine and chlorine, such as but not limited to $^{2}H$, $^{3}H$, $^{13}C$, $^{14}C$, $^{15}N$ , $^{18}O$, $^{17}O$, $^{31}P$, $^{32}P$, $^{35}S$, $^{18}F$ and $^{36}Cl$ respectively. The isotope-labeled compounds of the present invention can generally be prepared by conventional techniques known to those skilled in the art or by methods similar to those described in the attached examples using appropriate isotopically-labeled reagents instead of non-isotopically-labeled reagents. Such compounds have various potential uses, for example as standards and reagents in the determination of biological activity. In the case of stable isotopes, such compounds have the potential to advantageously alter biological, pharmacological or pharmacokinetic properties.

The term "prodrug" means that the compound of the present invention can be administered in the form of a prodrug. A prodrug refers to a derivative of the biologically active compound of the invention that is transformed into a derivative of the biologically active compound of the present invention under physiological conditions in the living body, for example, through oxidation, reduction, hydrolysis, etc. (each of them utilizes enzymes or is performed without the participation of enzymes). Examples of prodrugs are compounds as following: wherein the amino group in the compound of the present invention is acylated, alkylated or phosphorylated, such as eicosanylamino, alanylamino, pivaloyloxymethylamino, or wherein hydroxy is acylated, alkylated, phosphorylated or converted into borate, such as acetoxy, palmitoyloxy, pivaloyloxy, succinyloxy, fumaryloxy, alanyloxy, or wherein the carboxyl group is esterified or amidated, or wherein the sulfhydryl group forms a disulfide bridge with a carrier molecule, such as a peptide, that selectively delivers the drug to the target and/or to the cytosol of the cell.These compounds can be prepared from the compounds of the present invention according to known methods.

"Pharmaceutically acceptable salt" or "pharmaceutically acceptable salt" refers to salts made from pharmaceutically acceptable bases or acids, including inorganic bases or acids and organic bases or acids. When the compound of the present invention contains one or more acidic or basic groups, the present invention also includes their corresponding pharmaceutically acceptable salts. Therefore, the compounds of the invention containing acidic groups can exist in the form of salts and can be used according to the invention, for example as alkali metal salts, alkaline earth metal salts or as ammonium salts. More specific examples of such salts include sodium salts, potassium salts, calcium salts, magnesium salts, or salts with ammonia or organic amines, such as ethylamine, ethanolamine, triethanolamine, or amino acids. The compounds of the present invention containing basic groups can exist in the form of salts and can be used in the form of their addition salts with inorganic or organic acids according to the present invention. Examples of suitable acids include hydrochloric acid, hydrobromic acid, phosphoric acid, sulfuric acid, nitric acid, methanesulfonic acid, p-toluenesulfonic acid, naphthalenedisulfonic acid, oxalic acid, acetic acid, tartaric acid, lactic acid, salicylic acid, benzoic acid, formic acid, propanoic acid, pivalic acid, malonic acid, succinic acid, pimelic acid, fumaric acid, maleic acid, malic acid, sulfamic acid, phenylpropionic acid, gluconic acid, ascorbic acid, isonicotinic acid, citric acid, adipic acid and other acids known to those skilled in the art. If the compound of the present invention contains both acidic and basic groups in the molecule, the present invention also includes internal salts or betaines in addition to the salt forms mentioned. Each salt can be obtained by conventional methods known to those skilled in the art, for example, by contacting these with organic or inorganic acids or bases in a solvent or dispersant or by anion exchange or cation exchange with other salts.

The term "pharmaceutical composition" refers to composition containing one or more of the compounds described herein or their pharmaceutically acceptable salts, prodrugs, stable isotope derivatives, isomers and mixtures of the same, and other components such as pharmaceutically acceptable carriers and excipients. An object of the pharmaceutical compositions is to promote the administration of drugs to organisms, facilitate the absorption of active ingredients and thus exert biological activity.

Therefore, when referring to "compounds", "compounds of the present invention" or "compounds of the present invention" in this application, all forms of the compounds are included, such as pharmaceutically acceptable salts, prodrugs, stable isotopic derivatives, isomers and mixtures of the same.

As used herein, the term "tumor" includes benign tumors and malignant tumors (such as cancer).

As used herein, the term "therapeutically effective amount" refers to an amount of the compound of the present

invention that can effectively inhibit the function of IDO and/or treat or prevent the disease.

**Synthetic Method**

The present invention also provides a method for preparing the compound. The compound of general formula (I) of the present invention can be prepared by the following exemplary methods and examples, but these methods and examples should not be considered as limiting the scope of the present invention in any way. The compounds of the present invention can also be synthesized by synthetic techniques known to those skilled in the art, or a combination of methods known in the art and the methods of the present invention can be used. The product achieved in each step of the reaction is obtained by separation techniques known in the art, including but not limited to extraction, filtration, distillation, crystallization, chromatograpy and the like. The starting materials and chemical reagents required for the synthesis can be conventionally synthesized according to the literature (available on SciFinder) or be purchased.

The 3-indazolinone compound of the general formula (I) of the present invention can be synthesized according to the route described in method A: intermediate amine A1 undergoes reductive amination with o-nitrobenzaldehyde or undergoes substitution reaction with o-bromomethyl nitrobenzene to generate A2; the alcohol solution of A2 undergoes Davis-Beruit reaction in the presence of an alkali catalyst to obtain 2-substituted indazole A3; A3 is then hydrolyzed in acid solution to produce the target product 3-indazolinone compound A4.

Method A:

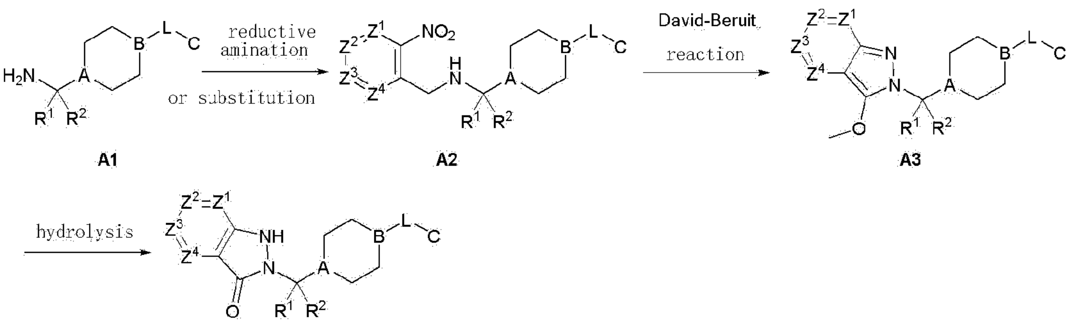


A1 A2 A3 A4

The intermediate amine A1 can be synthesized according to the related procedures as disclosed in patent application WO2017192844, or according to the route described in Method B: the starting material ketone B1 is reacted with trifluoromethanesulfonic anhydride under alkaline conditions to produce alkenyl trifluoromethanesulfonate B2; B2 is coupled with boronic acid ester or boronic acid $C\text{-}B(OR)_2$ through Suzuki coupling reaction to obtain $B_3$, which is reduced by hydrogenation to obtain B4; B4 is reduced by LAH to obtain an alcohol, which is further oxidized by Dess-Martin oxidant to produce an aldehyde B5; B5 is reacted with a Grignard reagent to obtain B6; B6 undergoes Mitsunobu reaction to produce B7; an amine A1 is obtained after final deprotection.

Method B :

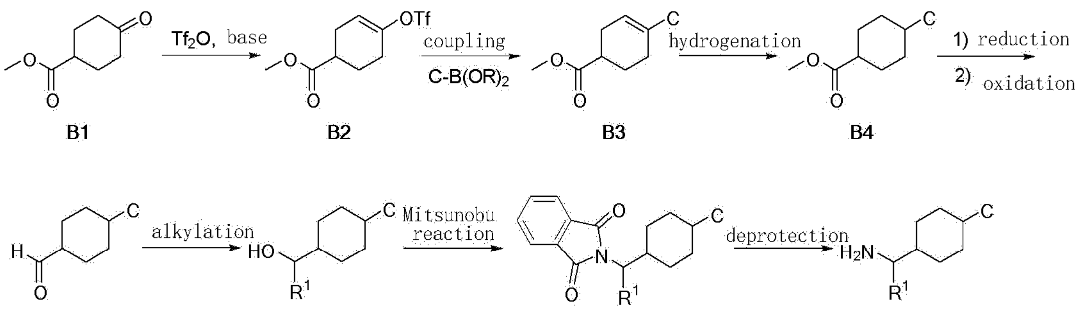


B1 B2 B3 B4

B5 B6 B7 $A_1$

## Examples

The structure of the compound is determined by nuclear magnetic resonance (NMR) or mass spectrometry (MS). NMR is determined with a NMR instrument Bruker ASCEND-400, the solvent used is deuterated dimethyl sulfoxide (DMSO-$d_6$), deuterated chloroform ($CDCl_3$), deuterated methanol ($CD_3OD$), the internal standard is tetramethyl silane (TMS), the chemical shifts are displayed in Chemical shifts (δ) are displayed in parts per million (ppm, $10^{-6}$).

MS is determined with an Agilent SQD (ESI) mass spectrometer (manufacturer: Agilent, model: 6120).

HPLC is determined with Agilent 1260 DAD high pressure liquid chromatograph (Poroshell120 EC-C18, 50×3.0 mm, 2.7 μm column) or Waters Arc high pressure liquid chromatograph (Sunfirc C18, 150×4.6 mm, 5 μm column)

The thin-layer silica gel plate used is a Qingdao Ocean GF254 silica gel plate. The size of the silica gel plate used in thin layer chromatography (TLC) is 0.15-0.2 mm, and the size of the silica gel plate used in separation and purification of product by thin layer chromatography is 0.4-0.5 mm. Qingdao Ocean 200-300 mesh silica gel is generally used as the support in the column chromatography.

The known starting raw materials of the present invention can be synthesized by or in accordance with methods known in the art, or can be purchased from companies such as ABCR GmbH&Co. KG, Acros Organics, Aldrich Chemical Company, Accela ChemBio Inc., Beijing Coupling Chemicals, etc.

In the examples, unless otherwise specified, the reactions are all carried out under an argon atmosphere or nitrogen atmosphere.

The argon atmosphere or nitrogen atmosphere means that the reaction flask is connected to an argon or nitrogen balloon with a volume of about 1 L.

The hydrogen atmosphere means that the reaction flask is connected to a hydrogen balloon with a volume of about 1 L.

In hydrogenation, the reaction is usually vacuumed and filled with hydrogen gas, and this procedure is repeated for 3 times.

The microwave reaction is carried out in a CEM Discover-SP type microwave reactor.

The reaction temperature is room temperature, and the temperature range is 20°C-30°C, unless otherwise specified.

An Agilent LC/MS instrument (1260/6120) is used in the monitor of the reaction progress in the examples. The progress of the reaction can also be monitored by thin layer chromatography (TLC). The system used in the developing agent includes A: dichloromethane and methanol system; B: petroleum ether and ethyl acetate system. The volume ratio of the solvent is adjusted according to the polarity of the compound.

The eluent system of column chromatography and the developing solvent system of thin layer chromatography used to purify the compound include A: dichloromethane and methanol system; B: petroleum ether and ethyl acetate system. The volume ratio of the solvent is adjusted according to the polarity of the compound, and a small amount of triethylamine and an acidic or alkaline reagent can also be added for adjustment, or other solvent systems can also be used. Waters mass spectrometry guided automatic preparation system (mass spectrometer detector: SQD2) is also used in the purification of compounds, for example, the reversed-phase high pressure column (XBridge-C18, 19×150 mm, 5 μm) is eluted by an appropriate acetonitrile/water (containing 0.1% trifluoroacetic acid or formic acid) or acetonitrile/water (containing 0.05% ammonia) gradient at a flow rate of 20 mL/min according to the polarity of the compound.

Examples 1 and 2

6-chloro-2-(((1r,4r)-4-(6-fluoroquinolin-4-yl)cyclohexyl)methyl)-1,2-dihydro-3H-indazol-3-one **1** and
6-chloro-2-(((1s,4s)-4-(6-fluoroquinolin-4-yl)cyclohexyl)methyl)-1,2-dihydro-3H-indazol-3-one **2**

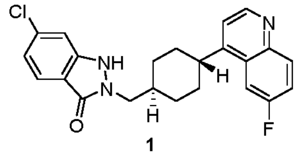
1

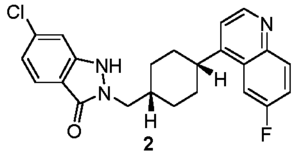
2

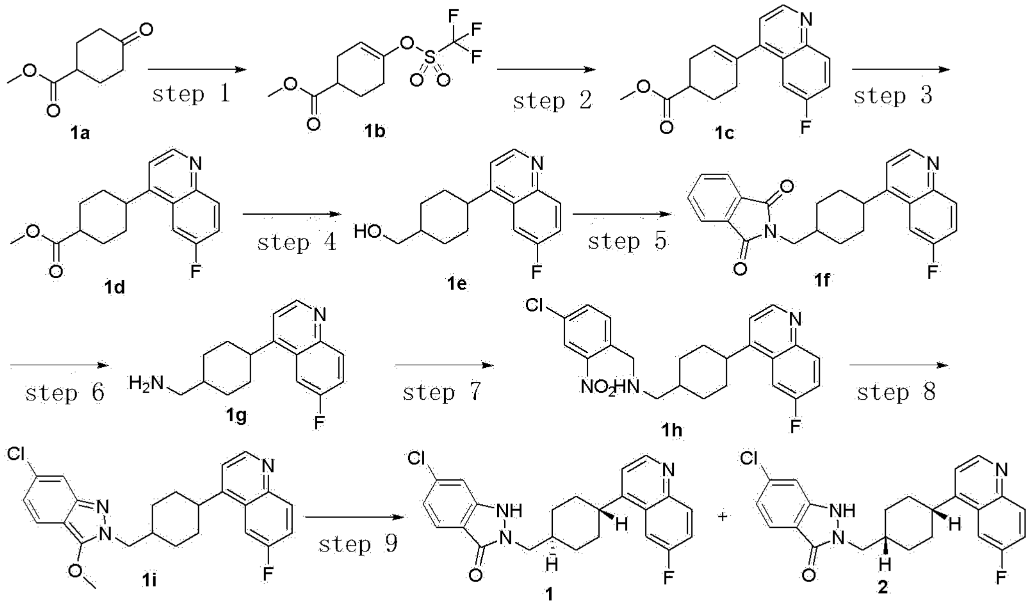


Step 1

methyl 4-(((trifluoromethyl)sulfonyl)oxy)cyclohex-3-ene-1-carboxylate

2,6-di-tert-butyl-4-methylpyridine (4.1 g, 20 mmol) was dissolved into dichloromethane (15 mL), then methyl 4-oxocyclohexane-1-carboxylate **1a**(1.80 g, 18 mmol) and trifluoromethanesulfonic anhydride (5.7 g, 20 mmol) were added sequentially. The reaction mixture was stirred at room temperature under argon atmosphere for 24 hours and then filtered. The filter cake was washed with ethyl acetate (30 mL×3). The organic phases were combined, washed sequentially with cold 1 N hydrochloric acid (50 mL) and saturated brine (50 mL) and dried over anhydrous sodium carbonate. After being filtered again, the solvent was removed from the filtrate under reduced pressure to obtain the target product methyl 4-(((trifluoromethyl)sulfonyl)oxy)cyclohex-3-ene-1-carboxylate **1b**(4.2 g, colorless oil), yield: 76%.
$^1$H NMR (400 MHz, $CDCl_3$) δ 5.82 - 5.68 (m, 1H), 3.70 (s, 3H), 2.60 (ddd, *J* = 10.5, 7.0, 3.3 Hz, 1H), 2.48 - 2.35 (m, 4H), 2.13 (ddd, *J* = 8.9, 4.1, 1.4 Hz, 1H), 1.93 (ddd, *J* = 6.9, 4.7, 2.6 Hz, 1H).

Step 2

methyl 4-(6-fluoroquinolin-4-yl)cyclohex-3-ene-1-carboxylate

A mixture of methyl 4-(((trifluoromethyl)sulfonyl)oxy)cyclohex-3-ene-1-carboxylate **1b**(4.2 g, 14.6 mmol), (6-fluoroquinolin-4-yl) boronic acid(2.78 g, 14.6 mmol), [1,1'-Bis(diphenylphosphino)ferrocene] palladium dichloride dichloromethane complex (1.19 g, 1.46 mmol), potassium carbonate (403 mg, 2.92 mmol), water (5 mL) and 1,4-dioxane (20 mL) was heat to 100°C under the protection of nitrogen, and stirring was continued for 2 hours. The reaction mixture was cooled to room temperature, concentrated under reduced pressure, and the residue was purified by silica gel column chromatography (petroleum ether/ethyl acetate = 20/1 to 1/1) to obtain the target product: methyl 4-(6-fluoroquinolin-4-yl)cyclohex-3-ene-1-carboxylate **1c** (3.32 g, pale yellow oil), yield: 80%.
MS m/z (ESI): 286[M+1]

Step 3

methyl 4-(6-fluoroquinolin-4-yl)cyclohexane-1-carboxylate

methyl 4-(6-fluoroquinolin-4-yl)cyclohex-3-ene-1-carboxylate **1c** (3.32 g, 11.67 mmol) was dissolved into methanol (50 mL), then 10% palladium on carbon (200 mg) was added, the reactant was stirred at room temperature for 2 hours under a hydrogen atmosphere. After filtration, the filtrate was concentrated under reduced pressure to obtain the target product: methyl 4-(6-fluoroquinolin-4-yl)cyclohexane-1-carboxylate **1d** (3.04 g, pale yellow solid), yield: 91%.
MS m/z (ESI): 288[M+1]

Step 4

(4-(6-fluoroquinolin-4-yl)cyclohexyl)methanol

methyl 4-(6-fluoroquinolin-4-yl)cyclohexane-1-carboxylate **1d** (1.2 g, 4.18 mmol) was dissolved into anhydrous tetrahydrofuran (20 mL), then lithium aluminum hydride (190 mg, 5 mmol) is added. After stirred at room temperature for 1 hour, water (0.5 mL), 15% sodium hydroxide solution (1 mL), water (0.5 mL) and anhydrous sodium sulfate (1 g) were added sequentially. The mixture was stirred for 15 minutes, then filtered. The filtrate was concentrated under reduced pressure to remove the solvent, and the target product (4-(6-fluoroquinolin-4-yl)cyclohexyl) methanol **1e** (810 mg, pale yellow solid) was achieved, yield: 80%.
MS m/z (ESI): 260[M+1]

Step 5

2-((4-(6-fluoroquinolin-4-yl)cyclohexyl)methyl)isoindoline-1,3-dione

(4-(6-fluoroquinolin-4-yl)cyclohexyl) methanol **1e** (500 mg, 1.93 mmol), phthalimide (312 mg, 2.12 mmol), triphenylphosphine (757 mg, 2.89 mmol) and tetrahydrofuran (20 mL) were mixed under nitrogen atmosphere, then diisopropyl azodicarboxylate (584 mg, 2.89 mmol) was added. After stirring for 5 hours at room temperature, the solvent was removed under reduced pressure, and the residue was purified by silica gel column chromatography (petroleum ether/ethyl acetate = 1/1) to obtain the target product 2-((4-(6-fluoroquinolin-4-yl)cyclohexyl)methyl)isoindoline-1,3-dione **1f** (400 mg, white solid), yield: 53%.
MS m/z (ESI): 389[M+1]

Step 6

(4-(6-fluoroquinolin-4-yl)cyclohexyl)methylamine

2-((4-(6-fluoroquinolin-4-yl)cyclohexyl)methyl)isoindoline-1,3-dione **1f** (510 mg, 1.31 mmol) was dissolved into ethanol (20 mL) and hydrazine hydrate (1 mL) was added, then the mixture was heated to 50 °C and stirred for 5 hours. After cooled to room temperature, the solvent was removed under reduced pressure, and the residue was purified by reverse phase preparative high performance liquid chromatography to obtain the target product (4-(6-fluoroquinolin-4-yl)cyclohexyl) methylamine **1g** (300 mg, white solid), yield: 80%.
MS m/z (ESI): 259[M+1]

Step 7

N-(4-chloro-2-nitrobenzyl)-1-(4-(6-fluoroquinolin-4-yl)cyclohexyl)met hanamine

(4-(6-fluoroquinolin-4-yl)cyclohexyl) methylamine 1g (260 mg, 1.0 mmol) and 2-nitro-4-chlorobenzaldehyde (190 mg, 1 mmol) were dissolved into methanol (10 mL), then sodium borohydride (94 mg, 1.5 mmol) was added. The reaction mixture was heated to 70°C and stirred for 1.5 hours. After cooled to room temperature, the solvent was removed under reduced pressure, and the residue was purified by silica gel column chromatography (petroleum ether/ethyl acetate = 25/1 to 2/1) to obtain the target product N-(4-chloro-2-nitrobenzyl)-1-(4-(6-fluoroquinolin-4-yl) cyclohexyl)methanamine **1h** (404 mg, white solid), yield: 94%.
MS m/z (ESI): 428[M+1]

Step 8

4-(4-((6-chloro-3-methoxy-2H-indazol-2-yl)methyl)cyclohexyl)-6-fluor oquinoline

N-(4-chloro-2-nitrobenzyl)-1-(4-(6-fluoroquinolin-4-yl)cyclohexyl)methan amine **1h** (0.6 g, 1.4 mmol), methanol (15 mL) and water (15 mL) were mixed, potassium hydroxide (393 mg, 7 mmol) was added, and the mixture was heated to 60°C and stirred for 24 hours. Water (50 mL) was added, then methanol was removed under reduced pressure, and the residue was extracted with dichloromethane (50 mL×2). The organic phases were combined and dried over anhydrous sodium sulfate. After filtration, the filtrate was concentrated under reduced pressure to obtain the target product 4-(4-((6-chloro-3-methoxy-2H-indazol-2-yl)methyl)cyclohexyl)-6-fluoroquinoline **1i** (550 mg, white solid), yield: 92%.
MS m/z (ESI): 424[M+1]

Step 9

6-chloro-2-(((1r,4r)-4-(6-fluoroquinolin-4-yl)cyclohexyl)methyl)-1,2-dihyd ro-3H-indazol-3-one **1** and 6-chloro-2-(((1s,4s)-4-(6-fluoroquinolin-4-yl)cyclohexyl)methyl)-1,2-dihyd ro-3H-indazol-3-one **2**

4-(4-((6-chloro-3-methoxy-2H-indazol-2-yl)methyl)cyclohexyl)-6-fluoroqu inoline **1i** (350 mg, 0.83 mmol) was dissolved into methanol (4 mL), then concentrated sulfuric acid (0.25 mL) was added. After that, the mixture was heated to 100°C in a microwave reactor and stirred overnight. After cooled to room temperature, the mixture was adjusted to pH = 7 with saturated sodium bicarbonate solution, and then extracted with ethyl acetate (25 mL×3). The organic phases were combined, the solvent was removed under reduced pressure, and the residue was purified by reverse-phase high performance liquid chromatography to obtain the target product 6-chloro-2-(((1r,4r)-4-(6-fluoroquinolin-4-yl)cyclohexyl)methyl)-1,2-dihyd ro-3H-indazol-3-one **1**(194 mg, white solid), yield: 51%; and 6-chloro-2-(((1s,4s)-4-(6-fluoroquinolin-4-yl)cyclohexyl)methyl)-1,2-dihyd ro-3H-indazol-3-one **2**(95 mg, white solid), yield: 25%.

6-chloro-2-(((1r,4r)-4-(6-fluoroquinolin-4-yl)cyclohexyl)methyl)-1,2-dihyd ro-3H-indazol-3-one **1**
MS m/z (ESI): 410[M+1]
$^1$H NMR (400 MHz, $CD_3OD$) δ 9.07 (d, *J* = 5.9 Hz, 1H), 8.32 (ddd, *J* = 14.2, 9.6, 3.7 Hz, 2H), 8.07-7.88 (m, 2H), 7.73 (d, *J* = 8.4 Hz, 1H), 7.33 (d, *J* = 1.2 Hz,1H), 7.14 (dd, *J* = 8.4, 1.7 Hz, 1H), 3.92 (d, *J* = 7.3 Hz, 2H), 3.63 (ddd, *J* = 11.9, 9.0, 3.0 Hz, 1H), 2.16-2.07 (m, *J* = 21.9, 3H), 1.96-1.80 (m, 2H),1.73 (qd, *J* = 12.7, 2.9 Hz, 2H), 1.52 (qd, *J* = 12.8, 3.1 Hz, 2H);
6-chloro-2-(((1s,4s)-4-(6-fluoroquinolin-4-yl)cyclohexyl)methyl)-1,2-dihyd ro-3H-indazol-3-one **2**
MS m/z (ESI): 410[M+1]
$^1$H NMR (400 MHz, $CD_3OD$) δ 9.16 (d, *J* = 5.9 Hz, 1H), 8.43-8.30 (m, 2H), 8.23 (d, *J* = 5.9 Hz, 1H), 8.04 (ddd, *J* = 9.4, 7.9, 2.6 Hz, 1H), 7.77 (d, *J* = 8.5 Hz,1H), 7.40 (d, *J* = 1.4 Hz, 1H), 7.20 (dd, *J* = 8.5, 1.7 Hz, 1H), 4.27 (d, *J* = 8.3 Hz, 2H), 3.71 (dd, *J* = 12.6, 9.4 Hz, 1H), 2.56 (s, 1H), 2.11-1.85 (m, 3H), 1.77 (d, J= 12.3 Hz, 1H).

Examples 3 and 4

6-chloro-2-(1-((1r,4r)-4-(6-fluoroquinolin-4-yl)cyclohexyl)ethyl)-1,2-dihyd ro-3H-indazol-3-one **3** and 6-chloro-2-(1-((1s,4s)-4-(6-fluoroquinolin-4-yl)cyclohexyl)ethyl)-1,2-dihy dro-3H-indazol-3-one **4**

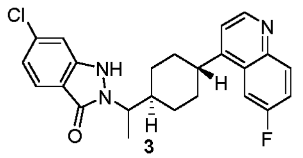
**3**

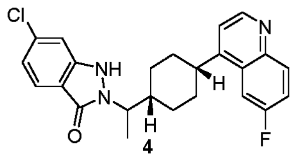
**4**

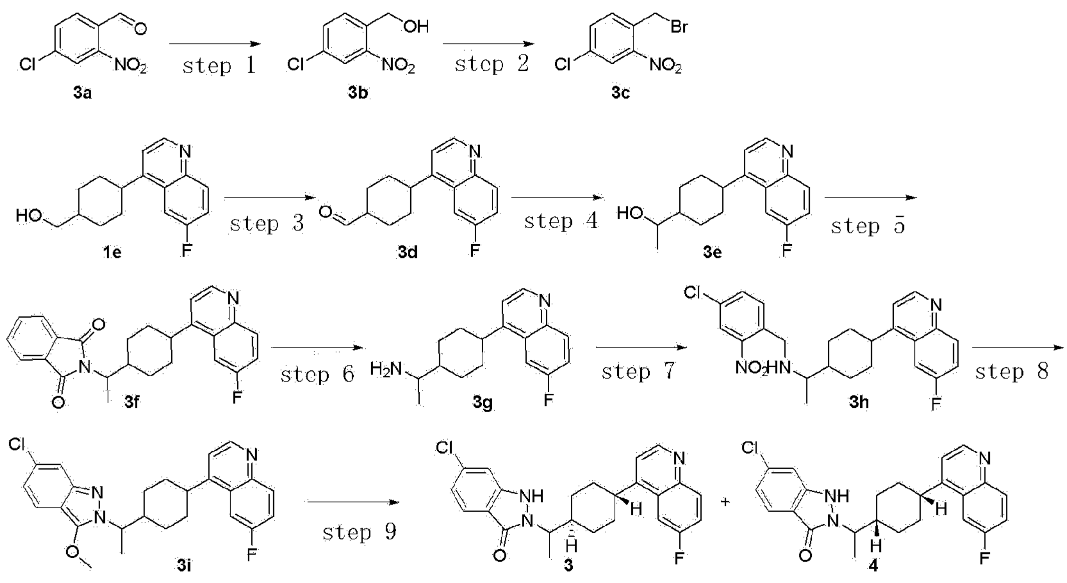


Step 1

2-nitro-4-chlorobenzyl alcohol

2-nitro-4-chlorobenzaldehyde **3a** (1.66 g, 8.95 mmol) was dissolved into methanol (20 mL), then sodium borohydride (509 mg, 13.4 mmol) was added. After stirred for 1 hour at room temperature, the reaction was quenched with water (1 mL), and the solvent was removed under reduced pressure. The residue was purified by silica gel column chromatography (petroleum ether/ethyl acetate = 10/1 to 1/1) to obtain the target product 2-nitro-4-chlorobenzyl alcohol **3b** (1.26 g, colorless oil), yield: 75%.
$^1$H NMR (400 MHz, $CDCl_3$) δ 8.10 (d, *J* = 2.1 Hz, 1H), 7.74 (d, *J* = 8.3 Hz, 1H), 7.64 (dd, *J* = 8.3, 2.1 Hz, 1H), 4.97 (s, 2H), 2.39 (s, 1H).

Step 2

1-bromomethyl-2-nitro-4-chlorobenzene

2-nitro-4-chlorobenzyl alcohol **3b** (1.26 g, 6.72 mmol) was dissolved into dichloromethane (20 mL), then carbon tetrabromide (4.05 g, 12.1 mmol) and triphenylphosphine (3.17 g, 12.1 mmol) were added sequentially. After stirred for 16 hours at room temperature, the solvent was removed under reduced pressure, and the residue was purified by silica gel column chromatography (petroleum ether/ethyl acetate = 100/0 to 4/1) to obtain the target product 1-bromomethyl-2-nitro-4-chlorobenzene **3c**(1.18 g, colorless oil), yield: 70%.
$^1$H NMR (400 MHz, $CDCl_3$) δ 8.05 (d, *J* = 2.1 Hz, 1H), 7.59 (dd, *J* = 8.3, 2.1 Hz, 1H), 7.53 (d, *J* = 8.3 Hz, 1H), 4.79 (s, 2H).

Step 3

4-(6-fluoroquinolin-4-yl)cyclohexane-1-carbaldehyde

(4-(6-fluoroquinolin-4-yl)cyclohexyl) methanol **le** (800 mg, 3.08 mmol) was dissolved into dichloromethane (20 mL), cooled to 0 °C, and Dess-Martin oxidant (1.5 g, 3.7 mmol) was added. After stirred for 2 hours at 0°C, the reaction mixture was filtered. The filtrate was concentrated under reduced pressure. The residue was purified by silica gel column chromatography (petroleum ether/ethyl acetate = 10/1 to 1/1) to obtain the target product 4-(6-fluoroquinolin-4-yl)cyclohexane-1-carbaldehyde **3d** (596 mg, colorless oil), yield: 75%.
MS m/z (ESI): 258[M+1]

Step 4

1-(4-(6-fluoroquinolin-4-yl)cyclohexyl)ethan-1-ol

4-(6-fluoroquinolin-4-yl)cyclohexane-1-carbaldehyde **3d** (1.67 g, 6.47 mmol) was dissolved into anhydrous tetrahydrofuran (20 mL), cooled to 0°C, then a solution of methyl magnesium chloride in tetrahydrofuran (3 M, 2.26 mL, 6.80 mmol) was added under nitrogen atmosphere. After stirred for 2 hours, the reaction was quenched with saturated ammonium chloride solution, and the solvent was removed under reduced pressure. The residue was purified by silica gel column chromatography (petroleum ether/ethyl acetate = 5/1 to 1/1) to obtain the target product 1-(4-(6-fluoroquinolin-4-yl)cyclohexyl)ethan-1-ol **3e** (1.2 g, colorless oil), yield: 70%.
MS m/z (ESI): 274[M+1]

Step 5

2-(1-(4-(6-fluoroquinolin-4-yl)cyclohexyl)ethyl)isoindoline-1,3-dione

1-(4-(6-fluoroquinolin-4-yl)cyclohexyl)ethan-1-ol **3e** (274 mg, 1.93 mmol), phthalimide (162 mg, 1.1 mmol) and triphenylphosphine (314 mg, 1.2 mmol) were mixed, and diisopropyl azodicarboxylate (243 mg, 1.2 mmol) was added under nitrogen atmosphere. After stirred at room temperature for 5 hours, the solvent was removed under reduced pressure. The residue was purified by silica gel column chromatography (petroleum ether/ethyl acetate = 10/1 to 1/1) to obtain the target product 2-(1-(4-(6-fluoroquinolin-4-yl)cyclohexyl)ethyl)isoindoline-1,3-dione **3f** (102 mg, white solid), yield: 25%
MS m/z (ESI): 403[M+1]

Step 6

1-(4-(6-fluoroquinolin-4-yl)cyclohexyl)ethan-1-amine

2-(1-(4-(6-fluoroquinolin-4-yl)cyclohexyl)ethyl)isoindoline-1,3-dione **3f** (600 mg, 1.5 mmol) was dissolved into ethanol (20 mL), hydrazine hydrate (1 mL) was added, then the reaction mixture was heated to 50°C and stirred for 5 hours. After cooled to room temperature, the solvent was removed under reduced pressure, and the residue was purified by reverse phase preparative high performance liquid chromatography to obtain the target product 1-(4-(6-fluoroquinolin-4-yl)cyclohexyl)ethan-1-amine **3g** (320 mg, white solid), yield: 74%.
MS m/z (ESI): 273[M+1]

Step 7

N-(4-chloro-2-nitrobenzyl)-1-(4-(6-fluoroquinolin-4-yl)cyclohexyl)etha n-1-amine

1-(4-(6-fluoroquinolin-4-yl)cyclohexyl)ethan-1-amine **3g** (272 mg, 1.0 mmol), 1-bromomethyl-2-nitro-4-chlorobenzene **3c** (250 mg, 1.0 mmol) and acetonitrile (20 mL) were mixed, then potassium carbonate (276 mg, 2 mmol) was added and heated to 70°C. After stirred for 2 hours at this temperature, the mixture was cooled to room temperature and then filtered. The solvent was removed from the filtrate under reduced pressure, and the residue was purified by silica gel column chromatography (petroleum ether/ethyl acetate = 10/1 to 1/1) to obtain the target product N-(4-chloro-2-nitrobenzyl)-1-(4-(6-fluoroquinolin-4-yl)cyclohexyl)ethan-1 -amine **3h** (265 mg, white solid), yield: 60%.
MS m/z (ESI): 442[M+1]

Step 8

4-(4-(1-(6-chloro-3-methoxy-2H-indazol-2-yl)ethyl)cyclohexyl)-6-fluor oquinoline

N-(4-chloro-2-nitrobenzyl)-1-(4-(6-fluoroquinolin-4-yl)cyclohexyl)ethan-1 -amine **3h** (265 mg, 0.6 mmol), methanol (15 mL) and water (15 mL) were mixed, potassium hydroxide (336 mg, 6 mmol) was added, then the mixture was heated to 60°C and stirred for 24 hours. Water (20 mL) was added, then methanol was removed under reduced pressure, and the residue was extracted with dichloromethane (30 mL×2). The organic phases were combined and dried over anhydrous sodium sulfate. After filtration, the solvent was removed from the filtrate under reduced pressure, and the residue was purified by silica gel column chromatography (petroleum ether/ethyl acetate = 10/1 to 1/1) to obtain the target product 4-(4-(1-(6-chloro-3-methoxy-2H-indazol-2-yl)ethyl)cyclohexyl)-6-fluoroquinoline **3i** (136 mg, white solid),

yield: 52%.
MS m/z (ESI): 438[M+1]

Step 9

6-chloro-2-(1-((1r,4r)-4-(6-fluoroquinolin-4-yl)cyclohexyl)ethyl)-1,2-dih ydro-3H-indazol-3-one **3**
and
6-chloro-2-(1-((1s,4s)-4-(6-fluoroquinolin-4-yl)cyclohexyl)ethyl)-1,2-di hydro-3H-indazol-3-one **4**

4-(4-(1-(6-chloro-3-methoxy-2H-indazol-2-yl)ethyl)cyclohexyl)-6-fluoroqu inoline **3i** (50 mg, 0.11 mmol) was dissolved into methanol (2 mL), concentrated sulfuric acid (0.1 mL) was added, then the mixture was heated to 100°C in a microwave reactor and stirred overnight. After cooled to room temperature, the mixture was adjusted to pH = 7 with saturated sodium bicarbonate solution, and extract with ethyl acetate (25 mL×3). The organic phases were combined, the solvent was removed under reduced pressure, and the residue was purified by reverse phase preparative high performance liquid chromatography to obtain the target product 6-chloro-2-(1-((1r,4r)-4-(6-fluoroquinolin-4-yl)cyclohexyl)ethyl)-1,2-dihydro-3H-indazol-3-one hydrochloride **3** (5 mg, white solid), yield: 10%;
and
6-chloro-2-(1-((1s,4s)-4-(6-fluoroquinolin-4-yl)cyclohexyl)ethyl)-1,2-dihydro-3H-indazol-3-one hydrochloride **4** (12 mg, white solid), yield: 24 %.

6-chloro-2-(1-((1r,4r)-4-(6-fluoroquinolin-4-yl)cyclohexyl)ethyl)-1,2-dihydro-3H-indazol-3-one hydrochloride **3**
MS m/z (ESI): 424[M+1]
$^1$H NMR (400 MHz, $CD_3OD$) δ 9.19 (s, 1H), 8.60 - 7.98 (m, 5H), 7.57 (s, 1H), 7.18 (s, 1H), 5.36 (s, 1H), 3.79 (s, 1H), 2.59 (d, J = 64.8 Hz, 1H), 2.21 (d, J = 20.4 Hz, 2H), 2.09 - 1.89 (m, 3H), 1.75 (d, J = 41.0 Hz, 3H), 1.31 (s, 3H);
6-chloro-2-(1-((1s,4s)-4-(6-fluoroquinolin-4-yl)cyclohexyl)ethyl)-1,2-dihydro-3H-indazol-3-one hydrochloride **4**
MS m/z (ESI): 424[M+1]
$^1$H NMR (400 MHz, $CD_3OD$) δ 9.19 (d, *J* = 5.9 Hz, 1H), 8.42 - 8.19 (m, 3H), 8.07 (ddd, *J* = 9.4, 7.9, 2.6 Hz, 1H), 7.77 (d, *J* = 8.5 Hz, 1H), 7.38 (d, *J* = 1.4 Hz,1H), 7.20 (dd, *J* = 8.5, 1.7 Hz, 1H), 5.14 (dd, *J* = 11.4, 6.7 Hz, 1H), 3.76 (s, 1H), 2.34 (d, *J* = 11.3 Hz, 1H), 2.22 - 1.92 (m, 5H), 1.88 - 1.72 (m, 2H), 1.55 (d, *J* = 6.7 Hz, 3H), 1.43 (d, *J* = 13.4 Hz, 1H).

Example 5

6-chloro-2-((R)-1-((1s,4S)-4-(2-methylpyridin-4-yl)cyclohexyl)ethyl)-1,2-d ihydro-3H-indazol-3-one

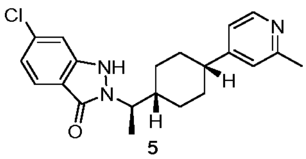

**5**

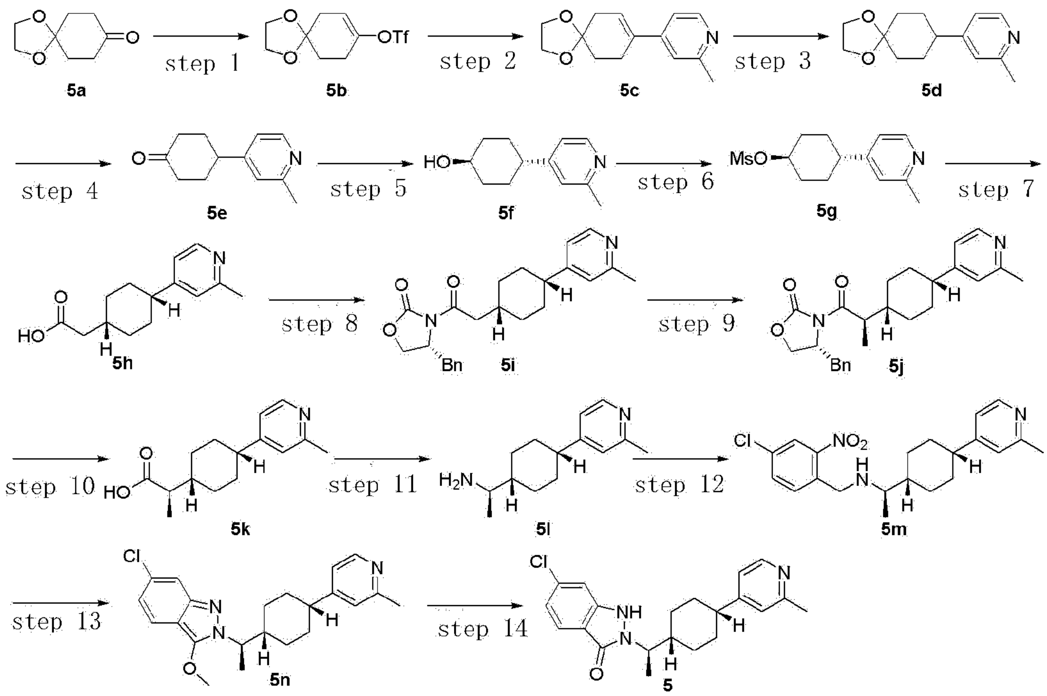


5a 5b 5c 5d 5e 5f 5g 5h 5i 5j 5k 5l 5m 5n 5

Step 1

1,4-Dioxaspiro[4.5]dec-7-en-8-yl trifluoromethanesulfonate

1,4-dioxaspiro[4.5]decane-8-one 5a (50 g, 320 mmol) was dissolved into anhydrous tetrahydrofuran (500 mL), cooled to -40°C under nitrogen atmosphere, then the solution of di(trimethylsilyl)sodium amide in tetrahydrofuran (2 M, 192 mL, 384 mmol) was added. After stirring for 1 hour at -40°C, the solution of N-phenylbis(trifluoromethanesulfonyl)imide (137 g, 384 mmol) in tetrahydrofuran (200 mL) was added gradually and stirring was continued for 1 hour. The reaction was quenched with saturated potassium hydrogen sulfate solution (50 mL) after it was finished. The mixture was filtered, and the filtrate was concentrated under reduced pressure. The residue was dissolved into a mixture of methyl tert-butyl ether (500 mL) and petroleum ether (500 mL) and filtered. The filtrate was washed with 30% sodium hydroxide solution (200 mL×3) and dried with anhydrous sodium sulfate. After filtration, the solvent was removed under reduced pressure to obtain the target product 1,4-dioxaspiro[4.5]dec-7-en-8-yl trifluoromethanesulfonate 5b (71.5 g, colorless oil), yield: 77 %.
$^1H$ NMR (400 MHz, $CDCl_3$) δ 5.66 (tt, *J* = 4.0, 1.3 Hz, 1H), 4.05 - 3.93 (m, 4H), 2.60 - 2.47 (m, 2H), 2.41 (dt, *J* = 4.0, 2.5 Hz, 2H), 1.90 (t, *J* = 6.6 Hz, 2H).

Step 2

2-methyl-4-(1,4-dioxaspiro[4.5]dec-7-en-8-yl)pyridine

The mixture of 1,4-dioxaspiro[4.5]dec-7-en-8-yl trifluoromethanesulfonate **5b** (4.0 g, 13.9 mmol), 2-methyl-4-pyridineboronic acid (1.58 g, 11.6 mmol)), [1,1'-bis(diphenylphosphino)ferrocene] palladium dichloride dichloromethane complex (422 mg, 0.57 mmol), potassium carbonate (2.39 g, 17.4 mmol), water (10 mL) and 1,4-dioxane (50 mL) was heated to 100 °C under the protection of nitrogen, and stirring was continued for 3 hours. The reaction mixture was cooled to room temperature, concentrated under reduced pressure, and the residue was purified by silica gel column chromatography (dichloromethane/methanol = 9/1) to obtain the target product 2-methyl-4-(1,4-dioxaspiro[4.5] dec-7-en-8-yl)pyridine **5c** (2.3 g, colorless oil), yield: 86%.
MS m/z (ESI): 232[M+1]

Step 3

2-methyl-4-(1,4-dioxaspiro[4.5]decan-8-yl)pyridine

2-methyl-4-(1,4-dioxaspiro[4.5]dec-7-en-8-yl)pyridine **5c** (2.3 g, 9.95 mmol) was dissolved into methanol (30 mL), then 10% Palladium on carbon (230 mg) was added, the mixture was stirred at room temperature for 2 hours under hydrogen atmosphere. The mixture was filtered and the filtrate was concentrated under reduced pressure to obtain the target product 2-methyl-4-(1,4-dioxaspiro[4.5]decan-8-yl)pyridine **5d** (2.3 g, colorless oil), yield: 99%.
MS m/z (ESI): 234[M+1]

Step 4

4-(2-methylpyridin-4-yl)cyclohexan-1-one

2-methyl-4-(1,4-dioxaspiro[4.5]decan-8-yl)pyridine **5d** (2.3 g, 9.87 mmol) was dissolved into tetrahydrofuran (30 mL), then 6 N hydrochloric acid (5 mL) was added. After heated to 50°C and stirred for 18 hours, the solvent was removed under reduced pressure, and the residue was neutralized with saturated sodium bicarbonate solution (5 mL), and extracted with ethyl acetate (50 mL×3). The organic phases were combined, the solvent was removed under reduced pressure, and the residue was purified by silica gel column chromatography (dichloromethane/methanol = 9/1) to obtain the target product 4-(2-methylpyridin-4-yl)cyclohexan-1-one **5e** (1.8 g, colorless oil), yield: 96%.
MS m/z (ESI): 190[M+1]

Step 5

(1r,4r)-4-(2-methylpyridin-4-yl)cyclohexan-1-ol

4-(2-methylpyridin-4-yl)cyclohexan-1-one **5e** (1.8 g, 9.52 mmol) was dissolved into isopropanol (30 mL), cooled to 0°C, and sodium borohydride (361 mg , 9.52 mmol) was added. After stirred for 1 hour at 0°C, the reaction was quenched with saturated ammonium chloride solution and filtered. The filtrate was concentrated under reduced pressure, and the residue was purified by silica gel column chromatography (dichloromethane/methanol = 9/1) to obtain the target product (1r,4r)-4-(2-methylpyridin-4-yl)cyclohexan-1-ol **5f** (1.6 g, colorless oil), yield: 88%.
MS m/z (ESI): 192[M+1]

Step 6

(1r,4r)-4-(2-methylpyridin-4-yl)cyclohexyl mesylate

(1r,4r)-4-(2-methylpyridin-4-yl)cyclohexan-1-ol **5f** (1.6 g, 8.37 mmol) was dissloved into anhydrous tetrahydrofuran (30 mL), cooled to 0°C, then triethylamine (1.27 g, 12.6 mmol) and methanesulfonyl chloride (1.06 g, 9.21 mmol) were added sequentially. After sitrred for 1 hour at 0°C, the reaction mixture was filtered. The filtrate was concentrated under reduced pressure, and the residue was purified by silica gel column chromatography (dichloromethane/methanol = 9/1) to obtain the target product (1r,4r)-4-(2-methylpyridin-4-yl)cyclohexyl mesylate **5g** (2.2 g, colorless oil), yield: 98%.
MS m/z (ESI): 270[M+1]

Step 7

2-((1s,4s)-4-(2-methylpyridin-4-yl)cyclohexyl)acetic acid

Di-tert-butyl malonate (5.54 g, 25.6 mmol) was dissolved into anhydrous tetrahydrofuran (30 mL), cooled to 0°C, then 60% sodium hydride (1.02 g, 25.5 mmol) was added. After stirred for 30 minutes, (1r,4r)-4-(2-methylpyridin-4-yl)cyclohexyl mesylate **5g** (2.2 g, 8.17 mmol) was added. The mixture was heated to 90°C and stirred for 18 hours. After cooled to room temperature, the mixture was adjusted to pH = 2 with 6 N hydrochloric acid, then heated to 100°C and stirred for 18 hours. After cooled to room temperature, the solvent was removed under reduced pressure, and the residue was purified by silica gel column chromatography (dichloromethane/methanol = 9/1) to obtain the target product 2-((1s,4s)-4-(2-methylpyridin-4)-yl)cyclohexyl)acetic acid **5h** (1.9 g, colorless oil), yield: 99%.
MS m/z (ESI): 234[M+1]

Step 8

(R)-4-benzyl-3-(2-((1s,4S)-4-(2-methylpyridin-4-yl)cyclohexyl)acetyl) oxazolidin-2-one

2-((1s,4s)-4-(2-methylpyridin-4-yl)cyclohexyl)acetic acid **5h** (1.9 g, 8.14 mmol) was dissolved into anhydrous tetrahydrofuran (20 mL), triethylamine (1.73 g, 17.16 mmol) was added, and the mixture was cooled to -78°C under nitrogen atmosphere, then pivaloyl chloride (1.13 g, 9.44 mmol) was added dropwise. After stirred for one hour at 0°C, a suspension was achieved for later use.

(R)-4-benzyloxazolidin-2-one (1.97 g, 11.15 mmol) was dissolved into anhydrous tetrahydrofuran (10 mL), cooled to -78°C, and the solution of n-butyl lithium in hexane (2.5 M, 4.4 mL, 11 mmol) was added dropwise under nitrogen atmosphere. After stirred for 15 minutes at -78°C, the reactant mixture was warmed gradually to 0°C and stirred for 15 minutes.

Then the resulting pale yellow solution was cooled to -78°C again for later use.

The above suspension was cooled to -78°C, then added into the light yellow solution that had been cooled to -78°C. The reaction mixture was gradually warmed to room temperature and stirring was continued for 3 hours. Saturated ammonium chloride solution (100 mL) was added to the reaction mixture and extracted with ethyl acetate (100 mL×3). The organic phases were combined, washed with saturated brine (20 mL×2). After dried over anhydrous sodium sulfate and filtered, the filtrate was concentrated under reduced pressure, and the residue was purified by silica gel column chromatography (dichloromethane/methanol = 9/1) to obtain the target product (R)-4-benzyl-3-(2-((1s,4S)-4-(2-methylpyridin-4-yl)cyclohexyl) acetyl)oxazolidin-2-one **5i** (3 g, colorless oil), yield: 91 %.
MS m/z (ESI): 393[M+1]

Step 9

(R)-4-benzyl-3-((R)-2-((1s,4S)-4-(2-methylpyridin-4-yl)cyclohexyl)pro pionyl)oxazolidin-2-one

(R)-4-benzyl-3-(2-((1s,4S)-4-(2-methylpyridin-4-yl)cyclohexyl)acetyl)oxaz olidin-2-one **5i** (3 g, 7.65 mmol) was dissolved into anhydrous tetrahydrofuran (30 mL), cooled to -50 °C, the solution of bis(trimethylsilyl)sodium amide in tetrahydrofuran (2 M, 7.7 mL, 15.4 mmol) was then added. After stirred for 30 minutes, iodomethane (1.63 g, 11.48 mmol) was added at this temperature and stirring was continued for 3 hours. After quenched with saturated ammonium chloride solution (10 mL), the reaction mixture was gradually warmed to room temperature and extracted with ethyl acetate (50 mL×3). The organic phases were combined, then washed with saturated brine (20 mL). After being dried with anhydrous sodium sulfate and filtered, the filtrate was concentrated under reduced pressure, and the residue was purified by silica gel column chromatography (dichloromethane/methanol = 9/1) to obtain the target product (R)-4-benzyl-3-((R)-2-((1s,4S)-4-(2-methylpyridin-4-yl) cyclohexyl)propionyl)oxazolidin-2-one **5j** (3.02 g, colorless oil), yield: 96%.
MS m/z (ESI): 407[M+1]

Step 10

(R)-2-((1s,4S)-4-(2-methylpyridin-4-yl)cyclohexyl)propionic acid

(R)-4-benzyl-3-((R)-2-((1s,4S)-4-(2-methylpyridin-4-yl)cyclohexyl)propio nyl)oxazolidin-2-one **5j** (3 g, 7.38 mmol), water (10 mL) and tetrahydrofuran (30 mL) were mixed, cooled to 0°C, then 35% hydrogen peroxide solution (2 mL) and lithium hydroxide monohydrate (266 mg, 11.03 mmol)were added sequentially. After gradually warmed to room temperature, stirring was continued for 1 hour. The reaction mixture was cooled to 0°C again, saturated sodium sulfite solution was added slowly to quench the reaction. The mixture was extract with ethyl acetate (50 mL×3). The organic phases were combined, the solvent was removed under reduced pressure, and the residue was purified by silica gel column chromatography (dichloromethane/methanol = 9/1), and further purified by reversed-phase high performance liquid chromatography to obtain the target product (R)-2-((1s,4S)-4-(2-methylpyridin-4-yl)cyclohexyl) Propionic acid **5k** (500 mg, colorless oil), yield: 27%.
MS m/z (ESI): 248[M+1]

Step 11

(R)-1-((1s,4S)-4-(2-methylpyridin-4-yl)cyclohexyl)ethan-1-amine

(R)-2-((1s,4S)-4-(2-methylpyridin-4-yl)cyclohexyl) propionic acid **5k** (200 mg, 0.8 mmol), diphenyl azidophosphate (241 mg, 0.87 mmol), triethylamine (98 mg, 0.97 mmol) and toluene (10 mL) were mixed, heated to 70°C and stirred for 3 hours. After cooled to room temperature, the solvent was removed under reduced pressure, the residue was dissolved into tetrahydrofuran (5 mL), then aqueous lithium hydroxide solution (1 N, 5 mL, 5 mmol) was added. After stirred for 1 hour at room temperature, the solvent was removed under reduced pressure, and the residue was purified by reverse-phase preparative high performance liquid chromatography to obtain the target product (R)-1-((1s,4S)-4-(2-methylpyridin-4-yl) cyclohexyl)ethan-1-amine **5l** (150 mg, yellow solid), yield: 86%.
MS m/z (ESI): 219[M+1]

Step 12

(R)-N-(4-chloro-2-nitrobenzyl)-1-((1s,4S)-4-(2-methylpyridin-4-yl)cycl ohexyl)ethan-1-amine

(R)-1-((1s,4S)-4-(2-methylpyridin-4-yl)cyclohexyl)ethan-1-amine **5l** (150 mg, 0.68 mmol), 1-bromomethyl-2-nitro-4-chlorobenzene **3c** (172 mg, 0.68 mmol) and acetonitrile (10 mL) were mixed, then potassium carbonate (190 mg, 1.37 mmol) was added and heated to 80°C. After stirred for 4 hours at this temperature, the reaction mixture was cooled to room temperature and filtered. The solvent was removed from the filtrate under reduced pressure to obtain the target product (R)-N-(4-chloro-2-nitrobenzyl)-1-((1s,4S)-4-(2-methylpyridin-4-yl)cyclohe xyl)ethan-1-amine **5m** (120 mg, yellow oil), yield: 46%.
MS m/z (ESI): 388[M+1]

Step 13

6-chloro-3-methoxy-2-((R)-1-((1s,4S)-4-(2-methylpyridin-4-yl)cyclohe xyl)ethyl)-2H-indazole

(R)-N-(4-chloro-2-nitrobenzyl)-1-((1s,4S)-4-(2-methylpyridin-4-yl)cyclohe xyl)ethan-1-amine **5m** (120 mg, 0.31 mmol), methanol (10 mL) and potassium hydroxide aqueous solution (0.5 N, 3.3 mL, 1.65 mmol) were mixed, then heated to reflux for 18 hours. After cooled to room temperature, the solvent was removed under reduced pressure, and the residue was purified by silica gel column chromatography (petroleum ether/ethyl acetate = 50/1 to 3/1) to obtain the target product 6-chloro-3-methoxy-2-((R)-1-((1s,4S)-4-(2-methylpyridin-4-yl)cyclohexyl) ethyl)-2H-indazole **5n** (100 mg, yellow solid), yield: 84 %.
MS m/z (ESI): 384[M+1]

Step 14

6-chloro-2-((R)-1-((1s,4S)-4-(2-methylpyridin-4-yl)cyclohexyl)ethyl)-1,2-d ihydro-3H-indazol-3-one

6-chloro-3-methoxy-2-((R)-1-((1s,4S)-4-(2-methylpyridin-4-yl)cyclohexyl) ethyl)-2H-indazole **5n**(100 mg, 0.26 mmol) was dissolved into methanol (10 mL), then concentrated sulfuric acid (0.12 mL) was added. The reaction mixture was then heated to 100°C in a microwave reactor and stirred for 5 hours. After cooled to room temperature, the mixture was adjusted to pH = 7 with saturated sodium bicarbonate solution, extract with ethyl acetate (25 mL×3). The organic phases were combined, the solvent was removed under reduced pressure, and the residue was purified by reverse phase preparative high performance liquid chromatography to obtain the target product 6-chloro-2-((R)-1-((1s,4S)-4-(2-methylpyridin-4-yl)cyclohexyl)ethyl)-1,2-d ihydro-3H-indazol-3-one hydrochloride **5** (32 mg, white solid), yield: 30%. MS m/z (ESI): 370[M+1]
$^1$H NMR (400 MHz, $CD_3OD$) δ 8.59 (d, *J* = 6.3 Hz, 1H), 7.93 (s, 1H), 7.88 (dd, *J* = 6.2, 1.4 Hz, 1H), 7.75 (d, *J* = 8.5 Hz, 1H), 7.38 - 7.32 (m, 1H), 7.18 (dd, *J* = 8.5, 1.7 Hz, 1H), 5.01 - 4.95 (m, 1H), 3.02 - 2.93(m, 1H), 2.80 (s, 3H), 2.26 - 2.18 (m, 1H), 2.04 - 1.84 (m, 5H), 1.72 - 1.64 (m, 1H), 1.62 - 1.55 (m, 1H), 1.50 (d, *J* = 8.0 Hz, 3H), 1.35 - 1.28 (m, 1H).

Example 6

6-chloro-2-((R)-1-((1s,4S)-4-(6-fluoroquinolin-4-yl)cyclohexyl)ethyl)-1,2-dihydro-3H-indazol-3-one

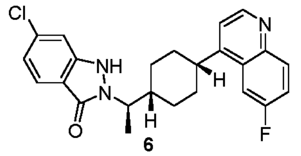

6

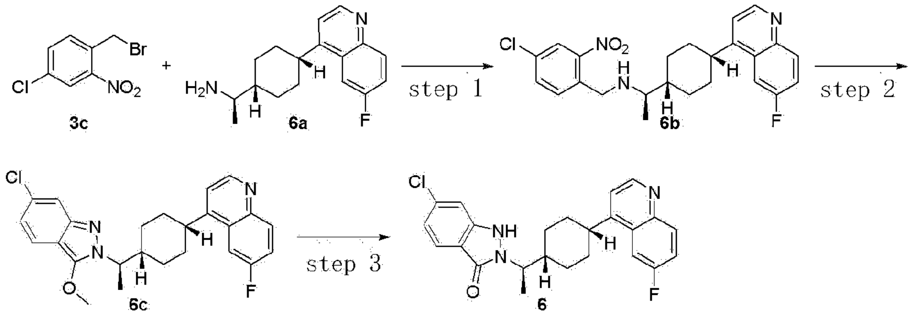


Step 1

(R)-N-(4-chloro-2-nitrobenzyl)-1-((1s,4S)-4-(6-fluoroquinolin-4-yl)cyc lohexyl)ethan-1-amine

1-bromomethyl-2-nitro-4-chlorobenzene 3c (175 mg, 0.7 mmol), potassium carbonate (193 mg, 1.4 mmol), (R)-1-((1s,4S)-4-(6-fluoroquinolin-4-yl)cyclohexyl)ethan-1-amine **6a** (190 mg, 0.7 mmol, the compound was synthesized according to the disclosure in the patent application WO2017192844A1) and acetonitrile (20 mL) were mixed and heated to 80°C. After stirred for 4 hours at this temperature, the mixture was cooled to room temperature, diluted with ethyl acetate (50 mL) and filtered. The solvent was removed from the filtrate under reduced pressure, and the residue was purified by silica gel column chromatography (petroleum ether/ethyl acetate = 100/1 to 2/1) to obtain the target product (R)-N-(4-chloro-2-nitrobenzyl)-1-((1s,4S)-4-(6-fluoroquinolin-4-yl)cyclohe xyl)ethan-1-amine **6b** (180 mg, yellow oil), yield: 58%.
MS m/z (ESI): 442[M+1]

Step 2

4-((1S,4s)-4-((R)-1-(6-chloro-3-methoxy-2H-indazol-2-yl)ethyl)cycloh exyl)-6-fluoroquinoline

(R)-N-(4-chloro-2-nitrobenzyl)-1-((1s,4S)-4-(6-fluoroquinolin-4-yl)cyclohe xyl)ethan-1-amine **6b** (180 mg, 0.4 mmol), methanol (20 mL) and potassium hydroxide aqueous solution (0.5 N, 4 mL, 2 mmol) were mixed, heated to reflux for 72 hours. After cooled to room temperature, the solvent was removed under reduced pressure, and the residue was purified by silica gel column chromatography (petroleum ether/ethyl acetate = 50/1 to 3/1) to obtain the target product 4-((1S,4s)-4-((R)-1-(6-chloro-3-methoxy-2H-indazol-2-yl)ethyl)cyclohexyl )-6-fluoroquinoline **6c** (150 mg, yellow solid), yield : 86%.
MS m/z (ESI): 438[M+1]

Step 3

6-chloro-2-((R)-1-((1s,4S)-4-(6-fluoroquinolin-4-yl)cyclohexyl)ethyl)-1,2-dihydro-3H-indazol-3-one

4-((1S,4s)-4-((R)-1-(6-chloro-3-methoxy-2H-indazol-2-yl)ethyl)cyclohexyl )-6-fluoroquinoline **6c** (150 mg, 0.34 mmol) was dissolved into methanol (10 mL), concentrated sulfuric acid (0.12 mL) was added, then the mixture was heated to 100°C and stirred for 5 hours. After cooled to room temperature, the residue was purified by reverse-phase preparative high performance liquid chromatography to obtain the target product 6-chloro-2-((R)-1-((1s,4S)-4-(6-fluoroquinolin-4-yl)cyclohexyl)ethyl)-1,2-dihydro-3H-indazol-3-one **6** (80 mg, green solid), yield: 56%.
MS m/z (ESI): 424[M+1]
$^1$H NMR (400 MHz, $CD_3OD$) $\delta$ 8.80 (d, *J* = 4.7 Hz, 1H), 8.09 (dd, *J* = 9.3, 5.6 Hz, 1H), 7.89 (dd, *J* = 10.7, 2.7 Hz, 1H), 7.74 (d, *J* = 8.4 Hz, 1H), 7.65 (d, *J* = 4.7 Hz, 1H), 7.59 (ddd, *J* = 9.2, 8.1, 2.8 Hz, 1H), 7.33 (d, *J* = 1.4 Hz, 1H), 7.15 (dd, *J* = 8.4, 1.7 Hz, 1H), 5.08 (dq, *J* = 13.5, 6.8 Hz, 1H), 3.50 - 3.37 (m, 1H), 2.29 (dd, *J* = 7.8, 3.4 Hz, 1H), 2.16 - 1.96 (m, 3H), 1.94 - 1.84 (m, 2H), 1.72 (ddd, *J* = 10.1, 7.0, 3.6 Hz, 2H), 1.51 (d, *J* = 6.8 Hz, 3H), 1.40 (d, *J* = 13.5 Hz, 1H).

Example 7

5-chloro-2-((R)-1-((1s,4S)-4-(6-fluoroquinolin-4-yl)cyclohexyl)ethyl)-1 ,2-dihydro-3H-indazol-3-one

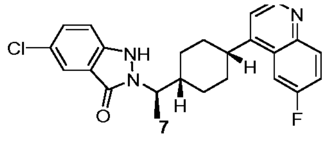

7

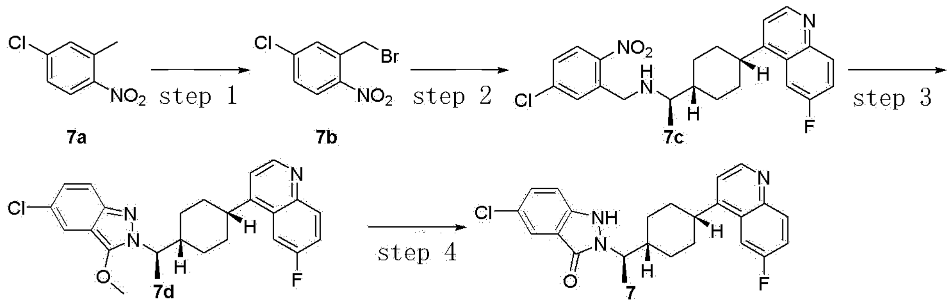


7a 7b 7c 7d 7

Step 1

2-bromomethyl-1-nitro-4-chlorobenzene

2-methyl-1-nitro-4-chlorobenzene **7a**(2.5g, 14.5mmol), N-bromosuccinimide (3.1 g, 17.5 mmol), azobisisobutyronitrile (237 mg, 1.45 mmol) and carbon tetrachloride (60 mL) were mixed and heated to reflux for 15 hours. After cooled to room temperature, the solvent was removed under reduced pressure, and the residue was purified by silica gel column chromatography (petroleum ether/ethyl acetate = 100/1 to 15/1) to obtain the target product 2-bromomethyl-1-nitro-4-chlorobenzene **7b** (170 mg, light yellow oil), yield: 5%.
$^1$H NMR (400 MHz, $CDCl_3$) δ 8.03 (d, *J* = 8.7 Hz, 1H), 7.58 (d, *J* = 2.3 Hz, 1H), 7.46 (dd, *J* = 8.7, 2.3 Hz, 1H), 4.80 (s, 2H).

Step 2

(R)-N-(5-chloro-2-nitrobenzyl)-1-((1s,4S)-4-(6-fluoroquinolin-4-yl)cyc lohexyl)ethan-1-amine

2-bromomethyl-1-nitro-4-chlorobenzene **7b** (170 mg, 0.68 mmol), potassium carbonate (187 mg, 1.36 mmol), (R)-1-((1s,4S)-4-(6-fluoroquinolin-4-yl)cyclohexyl)ethan-1-amine 6a (185 mg, 0.68 mmol) and acetonitrile (20 mL) were mixed and heated to 80°C. After stirred for 4 hours at this temperature, the mixture was cooled to room temperature, diluted with ethyl acetate (50 mL) and filtered. The solvent was removed from the filtrate under reduced pressure, and the residue was purified by silica gel column chromatography (petroleum ether/ethyl acetate = 100/1 to 2/1) to obtain the target product (R)-N-(5-chloro-2-nitrobenzyl)-1-((1s,4S)-4-(6-fluoroquinolin-4-yl)cyclohe xyl)ethan-1-amine **7c** (140 mg, yellow oil), yield: 46%.
MS m/z (ESI): 442[M+1]

Step3

4-((1S,4s)-4-((R)-1-(5-chloro-3-methoxy-2H-indazol-2-yl)ethyl)cycloh exyl)-6-fluoroquinoline

(R)-N-(5-chloro-2-nitrobenzyl)-1-((1s,4S)-4-(6-fluoroquinolin-4-yl)cyclohe xyl)ethan-1-amine **7c** (140 mg, 0.31 mmol), methanol (20 mL) and potassium hydroxide aqueous solution (0.5 N, 3.2 mL, 1.6 mmol) were mixed and heated to reflux for 72 hours. After cooled to room temperature, the solvent was removed under reduced pressure, and the residue was purified by silica gel column chromatography (petroleum ether/ethyl acetate = 50/1 to 3/1) to obtain the target product 4-((1S,4s)-4-((R)-1-(5-chloro-3-methoxy-2H-indazol-2-yl)ethyl)cyclohexyl )-6-fluoroquinoline **7d** (120 mg, yellow solid), yield : 88%.
MS m/z (ESI): 438[M+1]

Step4

5-chloro-2-((R)-1-((1s,4S)-4-(6-fluoroquinolin-4-yl)cyclohexyl)ethyl)-1,2-dihydro-3H-indazol-3-one

4-((1S,4s)-4-((R)-1-(5-chloro-3-methoxy-2H-indazol-2-yl)ethyl)cyclohexyl )-6-fluoroquinoline **7d** (120 mg, 0.27 mmol) was dissolved into methanol (10 mL), concentrated sulfuric acid (0.12 mL) was added, and the mixture was heated to 100°C and stirred for 5 hours. After cooled to room temperature, the residue was purified by reverse-phase high performance liquid chromatography to obtain the target product 5-chloro-2-((R)-1-((1s,4S)-4-(6-fluoroquinolin-4-yl)cyclohexyl)ethyl)-1,2-dihydro-3H-indazol-3-one 7 (47 mg, white solid), yield: 40%.
MS m/z (ESI): 424[M+1]
$^1$H NMR (400 MHz, $CD_3OD$) δ 8.81 (d, *J* = 4.7 Hz, 1H), 8.09 (dd, *J* = 9.3, 5.6 Hz, 1H), 7.89 (dd, *J* = 10.7, 2.7 Hz, 1H), 7.75 (d, *J* = 2.0 Hz, 1H), 7.65 (d, *J* = 4.7 Hz, 1H), 7.60 (ddd, *J* = 9.3, 8.2, 2.8 Hz, 1H), 7.54 (dd, *J* = 8.8, 2.1 Hz, 1H), 7.32 (d, *J* = 8.8 Hz, 1H), 5.09 (dt, *J* = 13.6, 6.8 Hz, 1H), 3.50 - 3.38 (m, 1H), 2.29 (dd, *J* = 7.9, 3.4 Hz, 1H), 2.16 - 1.99 (m, 3H), 1.91 (dd, *J* = 10.2, 6.1 Hz, 2H), 1.79 - 1.65 (m, 2H), 1.52 (d, *J* = 6.8 Hz, 3H), 1.39 (d, *J* = 15.7 Hz, 1H).

Example 8

6-chloro-2-((R)-1-((1s,4S)-4-(6-fluoroquinolin-4-yl)cyclohexyl)ethyl)-1,2-dihydro-3H-pyrazolo[4,3-b]pyridin-3-one

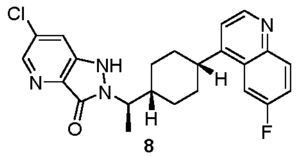
8

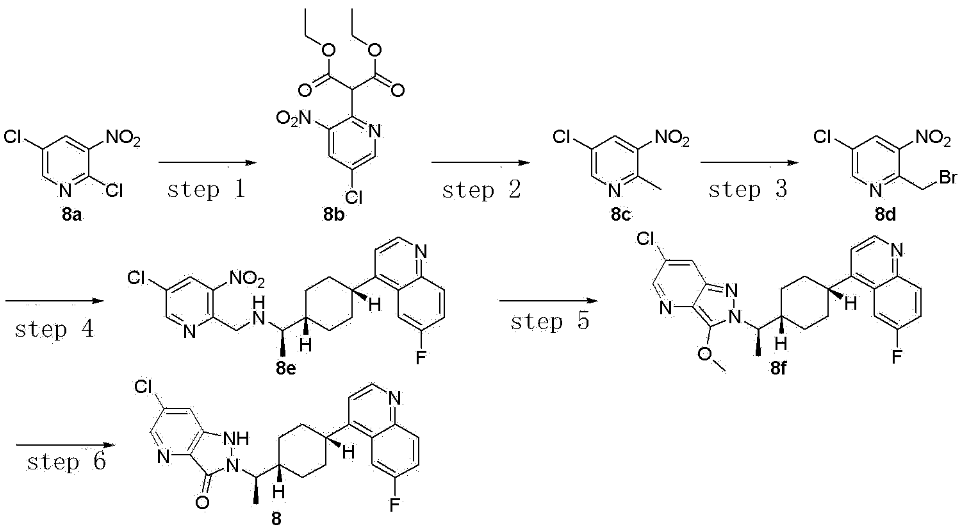

8a 8b 8c 8d 8e 8f 8

Step 1

Diethyl 2-(5-chloro-3-nitropyridin-2-yl)malonate

Diethyl malonate (4 g, 25 mmol), sodium hydride (60%, 1 g, 25 mmol) and N,N-dimethylformamide (15 mL) were mixed and stirred for 10 minutes, then the solution of 3-nitro-2,5-dichlorobenzene **8a** (2.5g, 13mmol) in N,N-dimethylformamide (5 mL) was added, and the mixture was heated to 40°C. After stirred for 2 hours at this temperature, water (40 mL) was added, and the mixture was adjusted to pH = 7-8 with 1 N hydrochloric acid and extracted with ethyl acetate (100 mL×2). The organic phases were combined, washed with saturated brine (20 mL), and dried over anhydrous sodium sulfate. After filtration, the filtrate was concentrated under reduced pressure, and the residue was purified by silica gel column chromatography (petroleum ether/ethyl acetate = 100/1 to 2/1) to obtain the target product diethyl 2-(5-chloro-3-nitropyridin-2-yl)malonate **8b** (3.2 g, pale yellow oil), yield: 78%.
MS m/z (ESI): 318[M+1]

Step 2

2-methyl-3-nitro-5-chloropyridine

Diethyl 2-(5-chloro-3-nitropyridin-2-yl)malonate **8b** (3.2 g, 10.1 mmol) and hydrochloric acid (5 N, 25 mL) were mixed and heated to reflux for 20 hours. After cooled to room temperature, the solvent was removed under reduced pressure, the residue was dissolved into methanol (50 mL) and adjusted to pH = 7-8 with saturated sodium bicarbonate solution, then the solvent was removed again under reduced pressure. The residue was purified by silica gel column chromatography (petroleum ether/ethyl acetate =100/1 to 1/1) to obtain the target product 2-methyl-3-nitro-5-chloropyridine **8c** (1.5 g, yellow oil), yield: 86%.
MS m/z (ESI): 173[M+1]

Step 3

2-(bromomethyl)-3-nitro-5-chloropyridine

2-methyl-3-nitro-5-chloropyridine **8c** (600 mg, 3.48 mmol), N-bromosuccinimide (743 mg, 4.18 mmol), azobisisobutyronitrile (57 mg , 0.35 mmol) and chlorobenzene (30 mL) were mixed and heated to reflux for 15 hours. After cooled to room temperature, the solvent was removed under reduced pressure, and the residue was purified by silica gel column chromatography (petroleum ether/ethyl acetate = 100/1 to 5/1) to obtain the target product 2-(bromomethyl)-3-nitro-5-chloropyridine 8d (300 mg, pale yellow oil), yield: 34%.
MS m/z (ESI): 251[M+1]

Step 4

(R)-N-((5-chloro-3-nitropyridin-2-yl)methyl)-1-((1s,4S)-4-(6-fluoroqui nolin-4-yl)cyclohexyl)ethan-1-amine

2-(bromomethyl)-3-nitro-5-chloropyridine **8d** (250 mg, 1 mmol), potassium carbonate (276 mg, 2 mmol), (R)-1-((1s,4S)-4-(6-fluoroquinolin-4-yl)cyclohexyl)ethan-1-amine **6a** (272 mg, 1 mmol) and acetonitrile (20 mL) were mixed and heated to 80°C. The mixture was stirred at this temperature for 4 hours, then it was cooled to room temperature, diluted with ethyl acetate (50 mL) and filtered. The solvent was removed from the filtrate under reduced pressure, and the residue was purified by silica gel column chromatography (petroleum ether/ethyl acetate = 100/1 to 2/1) to obtain the target product (R)-N-((5-chloro-3-nitropyridin-2-yl)methyl)-1-((1s,4S)-4-(6-fluoroquinolin-4-yl)cyclohexyl)ethan-1-amine **8e** (120 mg, yellow oil), yield: 27%.
MS m/z (ESI): 443[M+1]

Step 5

4-((1S,4s)-4-((R)-1-(6-chloro-3-methoxy-2H-pyrazolo[4,3-b]pyridin-2-yl)ethyl)cyclohexyl)-6-fluoroquinoline

(R)-N-((5-chloro-3-nitropyridin-2-yl)methyl)-1-((1s,4S)-4-(6-fluoroquinoli n-4-yl)cyclohexyl)ethan-1-amine **8e** (120 mg, 0.27 mmol), methanol (20 mL) and potassium hydroxide aqueous solution (0.5 N, 2.7 mL, 1.35 mmol) were mixed and heated to reflux for 72 hours. After cooled to room temperature, the solvent was removed under reduced pressure, and the residue was purified by silica gel column chromatography (petroleum ether/ethyl acetate = 50/1 to 3/1) to obtain the target product 4-((1S,4s)-4-((R)-1-(6-chloro-3-methoxy-2H-pyrazolo[4,3-b]pyridin-2-yl)ethyl)cyclohexyl)-6-fluoroquinoline **8f**(100 mg, yellow solid), yield: 84%.
MS m/z (ESI): 439[M+1]

Step 6

6-chloro-2-((R)-1-((1s,4S)-4-(6-fluoroquinolin-4-yl)cyclohexyl)ethyl)-1,2-dihydro-3H-pyrazolo[4,3-b]pyridin-3-one

4-((1S,4s)-4-((R)-1-(6-chloro-3-methoxy-2H-pyrazolo[4,3-b]pyridin-2-yl)e thyl)cyclohexyl)-6-fluoroquinoline **8f** (100 mg, 0.22 mmol) was dissolved into methanol (10 mL), concentrated sulfuric acid (0.12 mL) was added. The mixture was heated to 100°C and stirred for 5 hours. After cooled to room temperature, the residue was purified by reverse-phase preparative high performance liquid chromatography to obtain the target product 6-chloro-2-((R)-1-((1s,4S)-4-(6-fluoroquinolin-4-yl)cyclohexyl)ethyl)-1,2-dihydro-3H-pyrazolo[4,3-b]pyridin-3-one **8** (13mg, white solid), yield: 18%.

MS m/z (ESI): 425[M+1]
$^1$H NMR (400 MHz, $CD_3OD$) δ 8.86 (d, *J* = 4.6 Hz, 1H), 8.45 (s, 1H), 8.13 (dd, *J* = 9.2, 5.5 Hz, 1H), 7.96 (dd, *J* = 10.5, 2.2 Hz, 1H), 7.85 (d, *J* = 1.6 Hz, 1H), 7.74 (d, *J* = 4.8 Hz, 1H), 7.67 (dd, *J* = 11.9, 5.2 Hz, 1H), 5.17 (td, *J* = 13.4, 6.7 Hz, 1H), 3.49 (d, *J* = 7.1 Hz, 1H), 2.31 (d, *J* = 11.4 Hz, 1H), 2.07 (dd, *J* = 14.6, 10.6 Hz, 3H), 1.93 (d, *J* = 4.1 Hz, 2H), 1.81 - 1.67 (m, 2H), 1.54 (d, *J* = 6.7 Hz, 3H), 1.41 (d, *J* = 13.4 Hz, 1H).

Example 9

5-chloro-2-((R)-1-((1s,4S)-4-(6-fluoroquinolin-4-yl)cyclohexyl)ethyl)-1,2-dihydro-3H-pyrazolo[3,4-b]pyridin-3-one

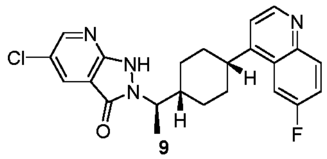
9

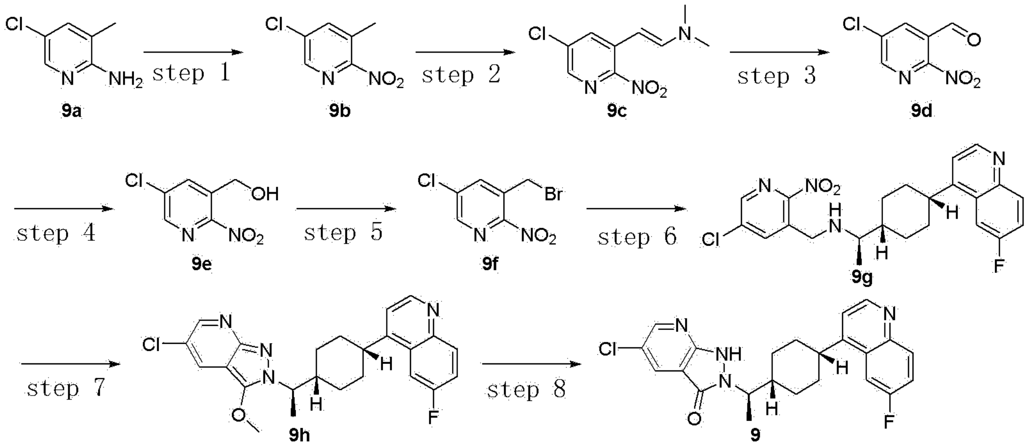

9a 9b 9c 9d 9e 9f 9g 9h 9

Step 1

5-chloro-3-methyl-2-nitropyridine

5-chloro-3-methylpyridin-2-amine **9a** (5.88 g, 41.4 mmol) was dissolved into concentrated sulfuric acid (50 mL) and cooled to -10°C, and the aqueous hydrogen peroxide solution (30%, 40 mL) was added dropwise. After the mixture was gradually warmed to room temperature, stirring was continued for 16 hours. When the reaction was finished, the mixture was poured into mixture of ice and water, and a white solid was precipitated out. The solid was filtered and dried to obtain the target product 5-chloro-3-methyl-2-nitropyridine **9b** (5.7 g, white solid), yield: 80%.
MS m/z (ESI): 173[M+1]

Step 2

(E)-2-(5-chloro-2-nitropyridin-3-yl)-N,N-dimethylethylen-1-amine

5-chloro-3-methyl-2-nitropyridine **9b** (5.16 g, 30 mmol) and N,N-dimethylformamide dimethyl acetal (4.28 g, 36 mmol) were dissolved into N,N-dimethylformamide (100 mL), the mixture was heated to 110°C and stirred overnight. After cooled to room temperature, the solvent was removed under reduced pressure, and the residue was purified by silica gel column chromatography (petroleum ether/ethyl acetate = 10/1 to 1/1) to obtain the target product (E)-2-(5-chloro-2-nitropyridin-3-yl)-N,N-dimethylethylen-1-amine **9c** (5.1 g, red solid), yield: 75%.
$^1$H NMR (400 MHz, $CD_3OD$) δ 8.18 (d, *J* = 2.2 Hz, 1H), 7.87 (d, *J* = 2.2 Hz, 1H), 7.44 (d, *J* = 13.3 Hz, 1H), 5.35 (d, *J* = 13.3 Hz, 1H), 2.99 (s, 6H).

Step 3

2-nitro-5-chloronicotinaldehyde

(E)-2-(5-chloro-2-nitropyridin-3-yl)-N,N-dimethylethylen-1-amine **9c** (2.28 g, 10 mmol) was dissolved into tetrahydrofuran (10 mL), then the solution of sodium periodate (3.21 g, 15 mmol) in water (15 mL) was added. The mixture was stirred overnight at room temperature and filtered. The filtrate was concentrated under reduced pressure. The residue was dispersed in tetrahydrofuran (100 mL) and stirred for 1 hour and filtered. The solvent was removed from the filtrate under reduced pressure to obtain the target product 2-nitro-5-chloronicotinaldehyde **9d** (954 mg, pale yellow solid), yield: 51%.
$^1$H NMR (400 MHz, $CDCl_3$) $\delta$ 10.28 (s, 1H), 8.65 (d, *J* = 2.4 Hz, 1H), 8.31 (d, *J* = 2.4 Hz, 1H).

Step 4

(5-chloro-2-nitropyridin-3-yl)methanol

2-nitro-5-chloronicotinaldehyde **9d** (144 mg, 0.77 mmol) was dissolved into methanol (20 mL), and sodium borohydride (58 mg, 1.54 mmol) was added at 0°C. After stirred for 2 hours at 0°C, the reaction was quenched with water (1 mL). The solvent was removed under reduced pressure, and the residue was purified by silica gel column chromatography (petroleum ether/ethyl acetate = 10/1 to 1/1) to obtain the target product (5-chloro-2-nitropyridin-3-yl)methanol **9e** (131 mg, colorless oil), yield: 75%.
MS m/z (ESI): 189[M+1]

Step 5

3-(bromomethyl)-5-chloro-2-nitropyridine

(5-chloro-2-nitropyridin-3-yl)methanol **9e** (131 mg, 0.696 mmol) was dissolved into dichloromethane (10 mL), carbon tetrabromide (415 mg, 1.25 mmol) and triphenylphosphine (328mg, 1.25mmol) were added sequentially. The reaction mixture was stirred for 16 hours at room temperature, then the solvent was removed under reduced pressure, and the residue was purified by silica gel column chromatography (petroleum ether/ethyl acetate = 100/0 to 4/1) to obtain the target product 3-(bromomethyl)-5-chloro-2-nitropyridine **9f** (81 mg, white solid), yield: 46%.
MS m/z (ESI): 251[M+1]

Step 6

(R)-N-((5-chloro-2-nitropyridin-3-yl)methyl)-1-((1s,4S)-4-(6-fluoroqui nolin-4-yl)cyclohexyl)ethan-1-amine

3-(bromomethyl)-5-chloro-2-nitropyridine **9f** (81 mg, 0.32 mmol), potassium carbonate (90 mg, 0.65 mmol), (R)-1-((1s,4S)-4-(6-fluoro quinolin-4-yl)cyclohexyl)ethan-1-amine **6a** (88 mg, 0.32 mmol) and acetonitrile (5 mL) were mixed and heated to 70°C, and stirred for 18 hours at this temperature. Then the mixture was cooled to room temperature, the solvent was removed under reduced pressure, and the residue was purified by silica gel column chromatography (petroleum ether/ethyl acetate = 100/1 to 2/1) to obtain the target product (R)-N-((5-chloro-2-nitro pyridin-3-yl)methyl)-1 -((1 s,4S)-4-(6-fluoroquinolin-4-yl)cyclohexyl)ethan-1-amine **9g** (75 mg, white solid), yield: 52%.
MS m/z (ESI): 443[M+1]

Step 7

4-((1S,4s)-4-((R)-1-(5-chloro-3-methoxy-2H-pyrazolo[3,4-b]pyridin-2-yl)ethyl)cyclohexyl)-6-fluoroquinoline

(R)-N-((5-chloro-2-nitropyridin-3-yl)methyl)-1-((1s,4S)-4-(6-fluoroquinoli n-4-yl)cyclohexyl) ethan-1-amine **9g** (75 mg, 0.17 mmol), methanol (2 mL) and aqueous potassium hydroxide solution (0.5 N, 1.6 mL, 0.8 mmol) were mixed, heated to 60°C and stirred for 24 hours. After cooled to room temperature, the solvent was removed under reduced pressure, and the residue was purified by silica gel column chromatography (petroleum ether/ethyl acetate = 50/1 to 3/1) to obtain the target product 4-((1S,4s)-4-((R)-1-(5-chloro-3-methoxy-2H-pyrazolo[3,4-b]pyridin-2-yl)ethyl)cyclohexyl)-6-fluoroquinoline **9h** (40 mg, white solid), yield: 55%.
MS m/z (ESI): 439[M+1]

Step 8

5-chloro-2-((R)-1-((1s,4S)-4-(6-fluoroquinolin-4-yl)cyclohexyl)ethyl)-1,2-dihydro-3H-pyrazolo[3 ,4-b]pyridin-3-one

4-((1S,4s)-4-((R)-1-(5-chloro-3-methoxy-2H-pyrazolo[3,4-b]pyridin-2-yl)e thyl)cyclohexyl)-6-fluoroquinoline **9h** (40 mg, 0.1 mmol) was dissolved into methanol (2 mL), concentrated sulfuric acid (0.12 mL) was added. The mixture was heated to 100°C and stirred for 5 hours. After cooled to room temperature, the residue was purified by reverse-phase high performance liquid chromatography to obtain the target product 5-chloro-2-((R)-1-((1s,4S)-4-(6-fluoroquinolin-4-yl)cyclohexyl)ethyl)-1,2-dihydro-3H-pyrazolo[3 ,4-b]pyridin-3-one hydrochloride **9** (10 mg, white solid), yield: 23%.
MS m/z (ESI): 425[M+1]
$^1$H NMR (400 MHz, $CD_3OD$) δ 9.17 (d, *J* = 5.7 Hz, 1H), 8.56 (d, *J* = 2.2 Hz, 1H), 8.36 (dt, *J* = 9.7, 3.7 Hz, 2H), 8.20 (dd, *J* = 12.6, 4.0 Hz, 2H), 8.10 -8.00 (m, 1H), 5.23-5.10 (m, 1H), 3.73 (d, *J* = 10.7 Hz, 1H), 2.35 (d, *J* = 10.9 Hz, 1H), 2.22-1.90 (m, 5H), 1.86-1.68 (m, 2H), 1.55 (d, *J* = 6.7 Hz, 3H), 1.44-1.30 (m, 1H).

Example 10

5,6-dichloro-2-((R)-1-((1s,4S)-4-(6-fluoroquinolin-4-yl)cyclohexyl)ethyl)-1,2-dihydro-3H-indazol-3-one

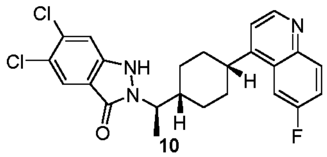

10

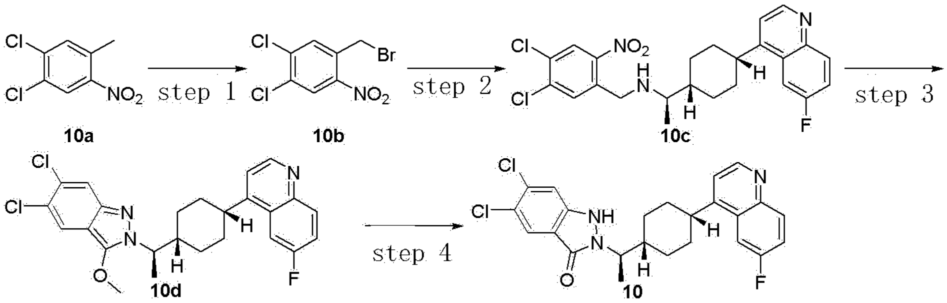


10a 10b 10c 10d 10

The compound in Example 10 was synthesized according to the same procedure as that of Example 7, except that in the first step, 1,2-dichloro-4-methyl-5-nitrobenzene **10a** was used instead of 2-methyl-1-nitro-4-chlorobenzene **7a.**
MS m/z (ESI): 458[M+1]
$^1$H NMR (400 MHz, $CD_3OD$) δ 9.18 (d, *J* = 5.9 Hz, 1H), 8.40 - 8.31 (m, 2H), 8.24 (d, *J* = 5.9 Hz, 1H), 8.09 - 8.02 (m, 1H), 7.90 (s, 1H), 7.54 (s, 1H), 5.11 (dq, *J* = 13.5, 6.7 Hz, 1H), 3.75 (t, *J* = 11.0 Hz, 1H), 2.37 - 2.28 (m, 1H), 2.16 - 1.94 (m, 5H), 1.86 - 1.73 (m, 2H), 1.54 (d, *J* = 6.7 Hz, 3H), 1.42 (d, *J* = 12.3 Hz, 1H).

Example 11

5-bromo-2-((R)-1-((1s,4S)-4-(6-fluoroquinolin-4-yl)cyclohexyl)ethyl)-1,2-dihydro-3H-indazol-3-one

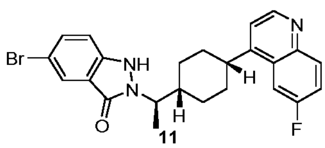

11

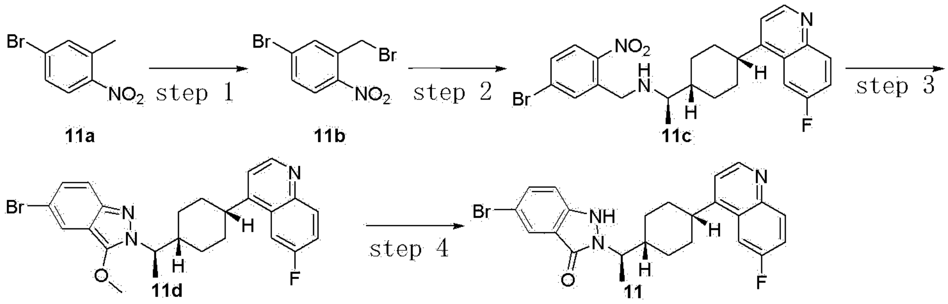


11a 11b 11c 11d 11

The compound in Example 11 was synthesized according to the same procedure as that of Example 7, except that in the first step, 2-methyl-1-nitro-4-bromobenzene **11a** was used instead of 2-methyl-1-nitro-4-chlorobenzene **7a.**
MS m/z (ESI): 468[M+1]
$^1$H NMR (400 MHz, $CD_3OD$) δ 8.81 (d, *J* = 4.7 Hz, 1H), 8.10 (dd, *J* = 9.3, 5.6 Hz, 1H), 7.90 (dd, *J* = 11.6, 2.1 Hz, 2H), 7.70 - 7.56 (m, 3H), 7.27 (d, *J* = 8.8 Hz, 1H), 5.10 (dd, *J* = 11.4, 6.7 Hz, 1H), 3.44 (d, *J* = 7.5 Hz, 1H), 2.30 (d, *J* = 11.1 Hz, 1H), 2.07 (dd, *J* = 26.4, 13.9 Hz, 3H), 1.91 (d, *J* = 4.1 Hz, 2H), 1.73 (dd, *J* = 18.0, 10.1 Hz, 2H), 1.52 (d, *J* = 6.8 Hz, 3H), 1.39 (d, *J* = 14.8 Hz, 1H).

Example 12

2-((R)-1-((1s,4S)-4-(6-fluoroquinolin-4-yl)cyclohexyl)ethyl)-3-oxo-2,3-dih ydro-1H-indazole-5-carbonitrile

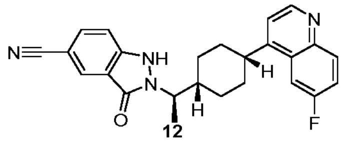

12

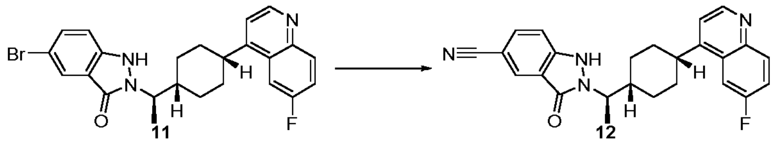

11 12

5-bromo-2-((R)-1-((1s,4S)-4-(6-fluoroquinolin-4-yl)cyclohexyl)ethyl)-1,2-dihydro-3H-indazol-3- one **11** (22 mg, 0.048 mmol), zinc cyanide (17 mg, 0.144 mmol), [1,1'-bis(diphenylphosphino)ferrocene]palladium dichloride (7 mg, 0.0096 mmol) and N,N-dimethylacetamide (3 mL) were mixed, deoxygenated, and heated to 130°C in a microwave reactor under nitrogen atmosphere and stirred for 1 hour. After cooled to room temperature, the solvent was removed under reduced pressure, and the residue was purified by reverse phase preparative high performance liquid chromatography to obtain the target product 2-((R)-1-((1 s,4S)-4-(6-fluoroquinolin-4-yl)cyclohexyl)ethyl)-3-oxo-2,3-dih ydro-1H-indazole-5-carbonitrile hydrochloride **12** (16 mg, pale yellow solid), yield: 76%.
MS m/z (ESI): 415[M+1]
$^1$H NMR (400 MHz, $CD_3OD$) δ 9.19 (d, *J* = 5.8 Hz, 1H), 8.43 - 8.33 (m, 2H), 8.25 (d, *J* = 5.9 Hz, 1H), 8.19 (d, *J* = 0.6 Hz, 1H), 8.10 - 8.01 (m, 1H), 7.82 (dd, *J* = 8.7, 1.5 Hz, 1H), 7.49 (d, *J* = 8.7 Hz, 1H), 5.19 (dq, *J* = 13.4, 6.7 Hz, 1H), 3.77 (t, *J* = 10.8 Hz, 1H), 2.37 (d, *J* = 11.2 Hz, 1H), 2.19 - 1.94 (m, 5H), 1.88 - 1.74 (m, 2H), 1.58 (d, *J* = 6.7 Hz, 3H), 1.40 (d, *J* = 12.6 Hz, 1H).

**IDO Intracellular activity inhibition test**

The effect of the compound of the present invention on the indoleamine 2,3-dioxygenase (IDO) activity in Hela cells induced by IFN-γ was evaluated by the Ehrlich method.
The principle underlined for this experiment is summarized as follows:
The expression of IDO in Hela cells is low in the absence of any induction. However, a certain concentration of IFN-γ can induce Hela cells to express IDO, so that it can catalyze the production of N-formylkynurenine from tryptophan. The produced N-formylkynurenine can be hydrolyzed by trichloroacetic acid to kynurenine, then a color reaction of the

achieved kynurenine with Ehrlich reagent can be used to detect the activity of IDO. The absorbance at 490 nm (OD490) is in proportion to the activity of IDO.

The compound was dissolved and diluted to 5 mM with DMSO (Sigma, catalog number: D5879), then serially diluted in 3 folds each time with DMSO to a minimum concentration of 2.29 μM. The diluent at each concentration point was further diluted in 50 folds with DMEM medium (ThermoFisher, catalog number: 11995073) free of FBS. If the $IC_{50}$ value of the compound was very low, the initial concentration of the compound could be lowered. Hela cells (ATCC, catalog number CCL-2) were cultured in DMEM complete medium containing 10% FBS (GBICO, catalog number 10099-141) and 100 U/mL penicillin streptomycin mixture (ThermoFisher, catalog number 15140122).When the cell coverage in the culture vessel reached 80-90%, 0.25% pancreatin (containing EDTA) (ThermoFisher, catalog number 25200056) was used to digest and blow off the cells, and the cells were grown in a 96-well plate (Corning, catalog number 3599), 30,000 cells per well (80 μL DMEM medium). The 96-well plate was then incubated in an incubator(37°C, 5% $CO_2$) overnight (18-20 hours).

10 μL of DMEM diluted compound and 10 μL of 500 ng/mL INF-γ were added to each well, and mixed gently. The 96-well plate was placed into an incubator(37°C, 5% $CO_2$) for further incubation. The mixture was taken out and centrifuged at 2000×g at room temperature for 5 minutes after 24 hours of incubation, the supernatant was transferred to the reaction plate (Sigma, catalog number CLS3695), one-twentieth of trichloroacetic acid (Sigma, product number T9159) was added, and incubated at 60°C after mixed well. 30 minutes later, the reaction plate was taken out and centrifuged at 2000×g at room temperature for 5 minutes, the supernatant was transferred to a clean reaction plate, equal volume of Ehrlich reagent was added, and the mixture was incubated at room temperature after mixed well. The OD490 of each well was determined 5 minutes later.

In the experiment, OD490 of the DMEM medium control group without IFN-γ was used as $OD490_{100\%inhibition}$; OD490 of the group in which the final concentration of IFN-γ in DMSO was 0.2% was used as $OD490_{0\%inhibition}$.

The percent inhibition of IDO activity in Hela cells by a compound can be calculated by the following formula:

$$\text{Inhibition percentage} = 100\text{-}100*(OD490_{compound}\text{-}OD490_{100\%inhibition})/(OD490_{0\%inhibition}\text{-}OD490_{100\%inhibition})$$

$IC_{50}$ of a compound was calculated by the following formula using 8 concentration points and XLfit (ID Business Solutions Ltd., UK) software

$$Y = \text{Bottom}+(\text{Top-Bottom})/(1+10\hat{}((\log IC_{50}\text{-}X)*\text{slope factor}))$$

where Y is inhibition percentage. Bottom is the bottom plateau of the S-type curve, Top is the top plateau of the S-type curve, X is the logarithm of the concentration of the tested compound, and slope factor is the slope coefficient of the curve.

The activity data of some representative example compounds are as follows:

| Number of the compound | $IC_{50}$ | Numberof the compound | $IC_{50}$ |
|---|---|---|---|
| 1 | B | 2 | A |
| 3 | B | 4 | A |
| 5 | A | 6 | A |
| 7 | A | 8 | A |
| 9 | A | 10 | A |
| 11 | A | | |

A<50nM; 50 nM ≤ B < 200 nM

The example compounds of the present invention respectively show a significant inhibitory effect on the activity of IDO in cells, preferably the $IC_{50}$ is less than 200 nM, more preferably the $IC_{50}$ is less than 50 nM.

## Claims

**1.** A compound as shown in general formula (I):

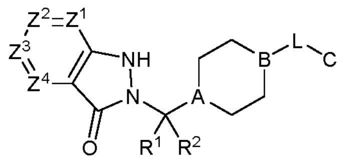

(I)

or a pharmaceutically acceptable salt, prodrug, stable isotope derivative, isomer thereof or mixture thereof, wherein:

$Z^1$, $Z^2$, $Z^3$ and $Z^4$ are each independently selected from N or $CR^3$, but $Z^1$, $Z^2$, $Z^3$ and $Z^4$ cannot be N at the same time;
$R^1$ and $R^2$ are each independently selected from H or optionally substituted $C_{1-4}$alkyl, $C_{3-6}$cycloalkyl or 4-7 membered heterocyclyl; or $R^1$ and $R^2$ may, together with the carbon atom to which they are attached, form a 3-7 membered ring optionally comprising a heteroatom selected from O, N and S;
A is N or $CR^4$;
B is N or $CR^5$;
L is a bond, -O- or $-CR^6R^7-$;
C is an optionally substituted 4-7 membered heterocyclyl, 6-10 membered aryl or 5-10 membered heteroaryl;
$R^3$ is independently selected from H, halogen, cyano, $-SF_5$, -OR, -SR, $-NR_2$, $-S(O)_mR$, $-S(O)_2NR_2$, $-N(R)S(O)_2R$, $-C(O)NR_2$, -N(R)C(O)R, or optionally substituted $C_{1-4}$alkyl, $C_{3-6}$cycloalkyl, 4-7 membered heterocyclyl, phenyl or 5-6 membered heteroaryl;
$R^4$ and $R^5$ are each independently selected from H, halogen, OH, or optionally substituted $C_{1-4}$ alkyl or $-O-C_{1-4}$ alkyl;
$R^6$ and $R^7$ are each independently selected from H or optionally substituted $C_{1-4}$alkyl;
R is independently selected from H or optionally substituted $C_{1-4}$ alkyl, $C_{3-6}$ cycloalkyl, 4-7 membered heterocyclyl, phenyl or 5-6 membered heteroaryl; the two Rs on the same nitrogen atom together with the nitrogen atom to which they are attached optionally form a 4-7 membered heterocyclic ring optionally containing additional heteroatom(s) selected from O, N and S;
m is 1 or 2.

2. The compound according to claim 1, or a pharmaceutically acceptable salt, prodrug, stable isotope derivative, isomer thereof or mixture thereof, wherein:

$Z^1$, $Z^2$, $Z^3$ and $Z^4$ are each independently selected from N or $CR^3$, but at most one of $Z^1$, $Z^2$, $Z^3$ and $Z^4$ is N;
$R^1$ and $R^2$ are each independently selected from H or optionally substituted $C_{1-4}$ alkyl;
A is N or $CR^4$;
B is N or $CR^5$;
L is a bond or -O-;
C is an 4-7 membered heterocyclyl, 6-10 membered aryl or 5-10 membered heteroaryl which is optionally substituted by halogen, cyano, $C_{1-4}$ alkyl or halogenated $C_{1-4}$ alkyl;
$R^3$ is independently selected from H, halogen, cyano, $-SF_5$, -OR, -SR, $-NR_2$, $-S(O)_mR$, $-S(O)_2NR_2$, $-N(R)S(O)_2R$, $-C(O)NR_2$, -N(R)C(O)R, or optionally substituted $C_{1-4}$ alkyl, $C_{3-6}$ cycloalkyl, 4-7 membered heterocyclyl, phenyl or 5-6 membered heteroaryl;
$R^4$ and $R^5$are each independently selected from H, halogen, OH, $C_{1-4}$ alkyl or $-O-C_{1-4}$ alkyl;
R is independently selected from H , or optionally substituted $C_{1-4}$ alkyl, $C_{3-6}$ cycloalkyl, 4-7 membered heterocyclyl, phenyl or 5-6 membered heteroaryl; the two Rs on the same nitrogen atom together with the nitrogen atom to which they are attached optionally form a 4-7 membered heterocyclic ring optionally containing additional heteroatom(s) selected from O, N and S;
m is 1 or 2.

3. The compound according to claim 1 or 2, or a pharmaceutically acceptable salt, prodrug, stable isotope derivative, isomer thereof or mixture thereof, wherein:

$Z^1$, $Z^2$, $Z^3$ and $Z^4$ are each independently selected from N or $CR^3$, but at most one of $Z^1$, $Z^2$, $Z^3$ and $Z^4$ is N;
$R^1$ and $R^2$ are each independently selected from H or $C_{1-4}$ alkyl;
A is N or CH;
B is N or CH;
L is a bond;

C is a 5-10 membered heteroaryl optionally substituted by halogen, cyano, $C_{1-4}$ alkyl or halogenated $C_{1-4}$ alkyl;
$R^3$ is independently selected from H, halogen, cyano, or optionally substituted $C_{1-4}$alkyl, -O-$C_{1-4}$alkyl, $C_{3-6}$cycloalkyl or 4-7 membered heterocyclyl;
the "optionally substituted" means substitution by a substituent selected from the group consisting of halogen, -CN, -OR', -NR'R", wherein R' and R" are each independently selected from H, C1-4 alkyl or $C_{3-7}$cycloalkyl.

**4.** The compound according to any one of the preceding claims, or a pharmaceutically acceptable salt, prodrug, stable isotope derivative, isomer thereof or mixture thereof, wherein:

$Z^1$ and $Z^4$ are each independently selected from N or CH, $Z^2$ and $Z^3$ are each independently selected from N or $CR^3$, but at most one of $Z^1$, $Z^2$, $Z^3$ and $Z^4$ is N;
$R^1$ and $R^2$ are each independently selected from H or $C_{1-4}$alkyl;
A is CH;
B is CH;
L is a bond;
C is a 5-10 membered heteroaryl optionally substituted by halogen or $C_{1-4}$ alkyl;
$R^3$ is H, halogen or cyano.

**5.** The compound according to any one of the preceding claims, which is a compound of the following general formula (IIa)-(IIc):

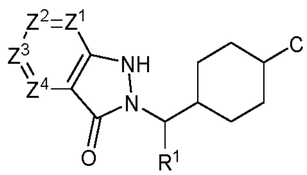 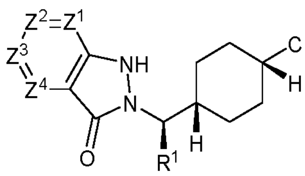 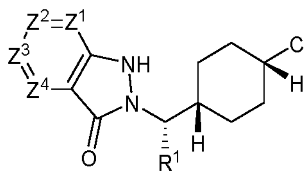

(IIa) (IIb) (IIc)

Wherein:

$R^1$ is $C_{1-4}$ alkyl;
$Z^1$, $Z^2$, $Z^3$ and $Z^4$ are as defined in claims 1-4.

**6.** The compound according to any one of the preceding claims, which is a compound of the following general formula (III):

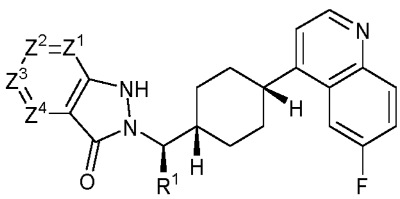 (III)

Wherein:

$R^1$ is $C_{1-4}$alkyl;
$Z^1$, $Z^2$, $Z^3$ and $Z^4$ are as defined in claims 1-4.

**7.** The compound according to any one of the preceding claims, or a pharmaceutically acceptable salt, prodrug, stable isotope derivative, isomer thereof or mixture thereof, the compound is selected from:

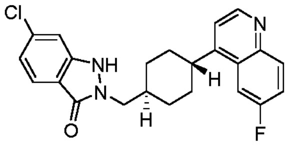,
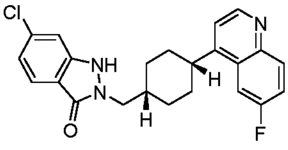,
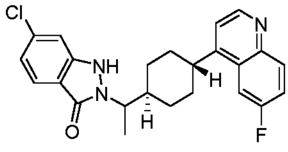,

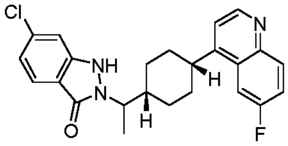,
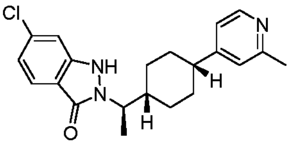,
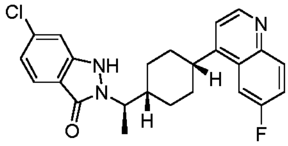,

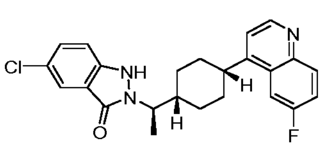,
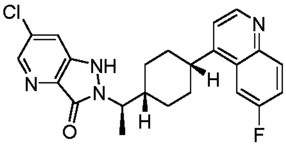,
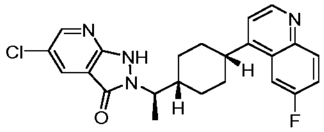,

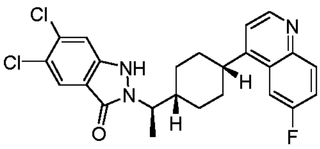,
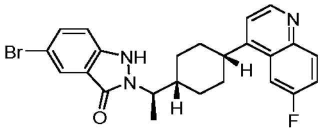,
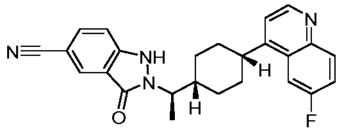.

**8.** A pharmaceutical composition, comprising a compound according to any one of claims 1 to 7 or a pharmaceutically acceptable salt, prodrug, stable isotope derivative, isomer thereof or mixture thereof, and a pharmaceutically acceptable carrier and excipient.

**9.** A pharmaceutical composition, comprising a compound according to any one of claims 1 to 7 or a pharmaceutically acceptable salt, prodrug, stable isotope derivative, isomer thereof and mixture of the same, and at least one additional medicament, wherein the at least one additional medicament is a chemotherapeutic agent, an immuno and/or inflammatory modulator, nerve-related disease modulators or an anti-infective.

**10.** The pharmaceutical composition according to claim 9, wherein the at least one additional medicament is an immune checkpoint inhibitor.

**11.** Use of a compound according to any one of claims 1 to 7 or a pharmaceutically acceptable salt, prodrug, stable isotope derivative, isomer thereof or mixture thereof, or use of a pharmaceutical composition according to any one of claims 8-10 in the manufacture of a medicament for treating and/or preventing IDO-mediated relative diseases, especially tumors, wherein the tumors are selected from prostate cancer, colon cancer, rectal cancer, pancreatic cancer, cervical cancer, stomach cancer, endometrial cancer, brain cancer, liver cancer, bladder cancer, ovarian cancer, testicular cancer, head cancer, neck cancer, skin cancer, mesothelial and endothelial carcinoma, lymphoma, leukemia, esophageal cancer, breast cancer, muscle cancer, connective tissue cancer, lung cancer, adrenal cancer, thyroid cancer, kidney cancer, bone cancer, glioblastoma, mesothelioma, sarcoma, chorionic carcinoma, basal cell carcinoma of the skin, or seminoma of the testis.

**INTERNATIONAL SEARCH REPORT**

International application No.
**PCT/CN2019/086258**

**A. CLASSIFICATION OF SUBJECT MATTER**

C07D 401/08(2006.01)i； A61K 31/4709(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNKI, VEN, CNABS, Caplus, Registry: 吲唑, 苯并吡唑, 喹啉, 酮, indazole, Pyrazol, oxo, structure search

**C. DOCUMENTS CONSIDERED TO BE RELEVANT**

| Category* | Citation of document, with indication, where appropriate, of the relevant passages | Relevant to claim No. |
|---|---|---|
| A | CN 101932325 A (NEWLINK GENETICS) 29 December 2010 (2010-12-29) see claim 144 | 1-11 |
| A | US 5173496 A (ICI PHARMA ET AL.) 22 December 1992 (1992-12-22) see entire document, especial claim 1, and description, p. 1, left column, lines 9-52 | 1-11 |
| A | XU, Guangwei et al. "A highly potent and selective inhibitor Roxyl-WL targeting IDO1 promotes immune response against melanoma." *Journal of Enzyme Inhibition and Medicinal Chemistry,* Vol. 33, No. 1, 22 January 2018 (2018-01-22), pages 1089-1094 and S1-S24 | 1-11 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **31 July 2019** | **16 August 2019** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/086258**

| Patent document cited in search report | | | Publication date (day/month/year) | Patent family member(s) | | | Publication date (day/month/year) |
|---|---|---|---|---|---|---|---|
| CN | 101932325 | A | 29 December 2010 | US | 2011053941 | A1 | 03 March 2011 |
| | | | | JP | 2011505379 | A | 24 February 2011 |
| | | | | JP | 2014159492 | A | 04 September 2014 |
| | | | | US | 2013289083 | A1 | 31 October 2013 |
| | | | | HK | 1143738 | A1 | 31 May 2013 |
| | | | | CA | 2707308 | A1 | 11 June 2009 |
| | | | | JP | 5583592 | B2 | 03 September 2014 |
| | | | | EP | 2227233 | A2 | 15 September 2010 |
| | | | | WO | 2009073620 | A2 | 11 June 2009 |
| | | | | US | 10047066 | B2 | 14 August 2018 |
| | | | | CA | 2707308 | C | 02 August 2016 |
| | | | | CN | 101932325 | B | 28 May 2014 |
| | | | | CA | 2932121 | A1 | 11 June 2009 |
| | | | | WO | 2009073620 | A9 | 25 March 2010 |
| | | | | EP | 2227233 | B1 | 13 February 2013 |
| US | 5173496 | A | 22 December 1992 | NO | 880182 | L | 20 July 1988 |
| | | | | NZ | 223194 | A | 26 March 1991 |
| | | | | NO | 880182 | A | 20 July 1988 |
| | | | | EP | 0284174 | A1 | 28 September 1988 |
| | | | | FI | 880195 | A | 20 July 1988 |
| | | | | IL | 84944 | A | 16 February 1992 |
| | | | | DE | 3873127 | T2 | 03 December 1992 |
| | | | | JP | S63253069 | A | 20 October 1988 |
| | | | | DK | 22888 | A | 20 July 1988 |
| | | | | AU | 8317587 | A | 21 July 1988 |
| | | | | AU | 606112 | B2 | 31 January 1991 |
| | | | | EP | 0284174 | B1 | 29 July 1992 |
| | | | | NO | 880182 | D0 | 18 January 1988 |
| | | | | DE | 3873127 | D1 | 03 September 1992 |
| | | | | FI | 880195 | A0 | 18 January 1988 |
| | | | | DK | 22888 | D0 | 19 January 1988 |
| | | | | PT | 86573 | A | 01 February 1988 |
| | | | | PT | 86573 | B | 31 December 1991 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006122150 A1
- WO 2011056652 A1
- WO 2013069765 A1
- WO 2014186035 A1
- WO 2015002918 A1
- WO 2016073738 A2
- WO 2016073770 A1
- WO 2016181348 A1
- WO 2016161960 A1
- WO 2017079669 A1
- WO 2017192844 A
- WO 2017192844 A1


### Non-patent literature cited in the description


- **KOBLISH.** *Mol. Cancer Ther.,* 2010, vol. 9, 489-98